# EUROPEAN PATENT SPECIFICATION

(11) **EP 3 472 196 B1**
(45) Date of publication and mention of the grant of the patent: **18.03.2026**
(21) Application number: 17731860.7
(22) Date of filing: 15.06.2017
(51) Int. Cl.: C07K 14/665, C07K 14/70, C12N 15/85, A61P 43/00, A61P 25/08

(54) **NEUROPEPTIDE-EXPRESSING VECTORS AND METHODS FOR THE TREATMENT OF EPILEPSY**
NEUROPEPTIDEXPRIMIERENDE VEKTOREN UND VERFAHREN ZUR BEHANDLUNG VON EPILEPSIE
VECTEURS EXPRIMANT DES NEUROPEPTIDES ET PROCÉDÉS POUR LE TRAITEMENT DE L'ÉPILEPSIE

(30) Priority: 16.06.2016 EP 16174827
(43) Date of publication of application: 24.04.2019
(73) Proprietor: Charité - Universitätsmedizin Berlin, 10117 Berlin (DE)
(72) Inventor: HEILBRONN, Regine, 12207 Berlin (DE); SCHWARZER, Christoph, 6020 Innsbruck (AT)
(74) Representative: Kilger, Ute
(86) International application number: PCT/EP2017/064692
(87) International publication number: WO 2017/216301

(56) References cited:
- WO-A2-03/093295
- WO-A2-2007/081792
- WO-A2-2007/112453
- WO-A2-2017/191274
- US-A1- 2013 116 165
- ANDREA ERNST: "Quantifizierung von Proneuropeptid- und Prohormonfragmenten im Blut und der Cerebrospinalfl�ssigkeit von Patienten mit Morbus Alzheimer", 1 October 2007 (2007-10-01), FU Berlin, XP055740874, Retrieved from the Internet <URL:https://refubium.fu-berlin.de/handle/fub188/4507> [retrieved on 20201016]
- VIVIAN HOOK ET AL: "Proteases for Processing Proneuropeptides into Peptide Neurotransmitters and Hormones", ANNUAL REVIEW OF PHARMACOLOGY AND TOXICOLOGY., vol. 48, no. 1, 1 February 2008 (2008-02-01), US, pages 393 - 423, XP055740875, ISSN: 0362-1642, DOI: 10.1146/annurev.pharmtox.48.113006.094812
- NAQVI T ET AL: "Structure-Activity Relationship Studies of Dynorphin A and Related Peptides", PEPTIDES, 1 January 1998 (1998-01-01), pages 1277 - 1292, XP093003624, Retrieved from the Internet <URL:https://doi.org/10.1016/S0196-9781(98)00042-4> [retrieved on 20221130]
- JOSHI ANAND A. ET AL: "Alanine scan of the opioid peptide dynorphin B amide", BIOPOLYMERS, vol. 108, no. 5, 2 May 2017 (2017-05-02), Hoboken, USA, pages e23026, XP093003938, ISSN: 0006-3525, Retrieved from the Internet <URL:https://api.wiley.com/onlinelibrary/tdm/v1/articles/10.1002%2Fbip.23026> DOI: 10.1002/bip.23026
- FOTI ET AL: "Novel AAV-mediated Therapeutic Strategies for Epilepsy", INTERNET CITATION, 1 October 2008 (2008-10-01), pages 1 - 183, XP002579373, Retrieved from the Internet <URL:http://gradworks.umi.com/33/04/3304308.html> [retrieved on 20100420]
- KOVAC STJEPANA ET AL: "Neuropeptides in epilepsy", NEUROPEPTIDES, vol. 47, no. 6, 2013, pages 467 - 475, XP028779393, ISSN: 0143-4179, DOI: 10.1016/J.NPEP.2013.10.015
- EDUARD SCHUNK ET AL: "Kappa opioid receptor activation blocks progressive neurodegeneration after kainic acid injection", HIPPOCAMPUS., 1 January 2010 (2010-01-01), US, pages n/a - n/a, XP055326874, ISSN: 1050-9631, DOI: 10.1002/hipo.20813
- JOHANNES BURTSCHER ET AL: "The Opioid System in Temporal Lobe Epilepsy: Functional Role and Therapeutic Potential", FRONTIERS IN MOLECULAR NEUROSCIENCE, vol. 10, 7 August 2017 (2017-08-07), XP055406616, DOI: 10.3389/fnmol.2017.00245

## Description

The present invention provides delivery vectors for transferring a nucleic acid sequence that encodes a pre-propeptide to a cell in vitro, ex vivo or in vivo. The present invention provides delivery vectors for use in delivering a nucleic acid sequence to a cell and delivery vectors for use in treating focal epilepsies.

With a prevalence of 1-2%, epilepsies belong to the most frequent neurological diseases worldwide (McNamara et al., 1999). Mesial temporal lobe epilepsy (mTLE) is the most frequent type of epilepsy in humans and is frequently induced by traumatic brain injury. Hippocampal sclerosis and accompanying neurological deficits are key features of mTLE (for review, see Engel et al., 2001). Despite the introduction of a plethora of antiepileptic drugs over the last few decades, the rate of drug-resistant epilepsies (30% to 70%) did not improve since the study of Coatsworth in 1971 (Coatsworth et al., 1971, Loscher et al., 2011). To date, surgical resection of the epileptogenic focus remains as the ultimate option for some of these patients. Even then, in certain subgroups of patients, less than 50% remain seizure free for at least one year after removal of the epileptic focus (Spencer et al., 2008).

Since the early 1980s, there has been evidence that opioids, namely dynorphin (Dyn), act as modulators of neuronal excitability in vitro (Henriksen et al., 1982, Siggins et al., 1986). In line with this, the deletion of the prodynorphin (pDyn) coding sequence in mice (Loacker et al., 2007) and low Dyn levels in humans due to mutations in the promoter region (Stogmann et al., 2002, Gambardella et al., 2003) are associated with increased vulnerability to the development of epilepsy. In most animal models of temporal lobe epilepsy (TLE; comprising epilepsies arising cortical = lateral TLE and mTLE), cortical and hippocampal pDyn expression is reduced after an initial, short peak of over-expression (for review, see (Simonato et al., 1996, Schwarzer et al., 2009). This is in line with most probably short-lived post-ictally increased pDyn mRNA in hippocampal granule cells (Pirker et al., 2009) accompanied by an overall reduction of Dyn-immunoreactivity in surgically removed tissue obtained from mTLE patients (de Lanerolle et al., 1997).

Dynorphins act preferentially on Kappa Opioid Receptors (KOR). Despite the reduction of endogenous Dyn, KOR remain available as drug target under epileptic conditions, and the application of KOR agonists can suppress experimental seizures (Tortella et al., 1988, Takahashi et al., 1990, Solbrig et al., 2006, Loacker et al., 2007, Zangrandi et al. 2016). Various selective KOR applied through different routes yielded time- and dose-dependent effects similar to those upon treatment with phenytoin or phenobarbital in models of epilepsy (for review, see (Simonato et al., 1996). We recently demonstrated that activation of KOR promotes the survival of hippocampal and amygdala neurons subsequent to the acute phase after unilateral injection of kainic acid in mice (Schunk et al., 2011).

WO 2007/081792 A2 describes certain compositions and methods related to improving pharmacological properties of bioactive compounds targeting nervous system.

XP002579373, describes AAV-mediated expression of galanin, and Neuropeptide Y either alone or in combination as therapeutic strategies for epilepsy.

US 2013/116165 A1 describes certain light-activated fusion proteins and their encoding polynucleotide, and certain methods of making and using the same.

WO 2007/112453 A2 describes certain compositions that can be used to treat, prevent, and modulate pain, inflammation, and metabolic processes in various organisms including plants and animals, formulation for administration to a human or a plant, and administration for topical or systemic use of the same.

WO 03/093295 A2 describes certain delivery vectors for transferring a nucleic acid sequence to a cell in vitro, ex vivo or in vivo, comprising a segment encoding a secretory signal peptide, as well as methods of transferring a nucleic acid of interest to a cell using the delivery vectors.

WO 2017/191274 A2 describes a certain RNA encoding a therapeutic protein, including for use as a medicament, compositions and kits comprising the RNA, as well as the use of the RNA, compositions or kits for increasing the expression of said encoded protein, in particular in gene therapy.

The object of the present invention is to provide delivery vectors for transferring a nucleic acid sequence encoding a pre-propeptide or a peptide comprising a signal sequence that enables the packing of said pro-peptide, e.g. a prodynorphin into vesicles, wherein the pro-peptide undergoes maturation and the active substance which is a dynorphin or variant of dynorphin is released upon a frequency of action potentials that exceeds a certain excitation threshold. The object of the present invention is, thus, to provide delivery vectors for transferring a nucleic acid sequence to a cell in vitro, ex vivo or in vivo. Object of the invention is in particular a vector-based therapy for treatment of focal epilepsies with pre-prodynorphin or dynorphin or variants thereof. The inventive delivery vectors comprising a nucleic acid encoding pre-prodynorphin or prodynorphin or dynorphin or variants thereof shall transduce neurons, release pre-prodynorphin or prodynorphin or dynorphin or variants thereof and thus provide activation of KOR in the epileptogenic focus, thereby inhibiting seizures.

Subject of the present invention is a delivery vector comprising a DNA sequence encoding pre-prodynorphin or pre-prodynorphin-variants and wherein said delivery vector drives expression of a pre-propeptide that enables the release of dynorphin or dynorphin-variants on demand, wherein said pre-propeptide is pre-prodynorphin or a pre-prodynorphin-variant and wherein said pre-propeptide comprise a signalpeptide that enables the packing of said pro-peptide into vesicles, and wherein said delivery vector leads to release-on-demand of dynorphins or dynorphin-variants, whereas said pre-prodynorphin or pre-prodynorphin-variants comprise at least one of the following sequences selected from the group:
a. Dyn A that is SEQ Id No. 7 (AA 207-223 of SEQ ID No. 1; ppDyn) or a variant thereof consisting of the first 13 AA (first from the N-terminal end that is YGGFLRRIRPKLK (SEQ ID No. 16)) or a variant thereof consisting of the first 8 AA (first from the N-terminal end that is YGGFLRRI (SEQ ID No. 17))
b. Dyn B that is SEQ ID No. 8 (AA 226-238 of SEQ ID No. 1; ppDyn)
c. leumorphin that is SEQ ID No. 9 (AA 226-254 of SEQ ID No. 1; ppDyn)
d. variants of Dyn A according to SEQ Id No.7 having an amino acid sequence identity of at least 60 % within the first 8 AA counted from the N-terminus of SEQ ID No. 7 (YGGFLRRI (SEQ ID No. 17)) i.e. having an amino acid sequence identity of at least 60 % within the sequence YGGFLRRI comprised in SEQ ID No. 7.
e. variants of Dyn B according to SEQ ID No. 8 having an amino acid sequence identity of at least 60 % within the first 8 AA counted from the N-terminus of SEQ ID No. 8 (YGGFLRRQ (SEQ ID No. 18)) i.e. having an amino acid sequence identity of at least 60 % within the sequence YGGFLRRQ comprised in SEQ ID No. 8.
f. variants of leumorphin according to SEQ ID No. 9 having an amino acid sequence identity of at least 60 % within the first 8 AA counted from the N-terminus of SEQ ID No. 9 (YGGFLRRQ (SEQ ID No. 18)), i.e. having an amino acid sequence identity of at least 60 % within the sequence YGGFLRRQ comprised in SEQ ID No. 9.

60 % sequence identity is defined as follows: 3 of the first 8 N-terminal amino acids may be removed or replaced by another amino acid. Percentage of sequence identity is calculated for the shortened peptide in case of truncated peptide variants. Introduction of additional amino acids are handled as gap in the original sequence, deletions are handled as gap in the modified peptide for calculation of sequence identity (YGGFLRRQ differs from YG-FLRRQ only by 1 AA, although now AA in positions 3, 4, 5, 6 and 7 are different). In any case a variant of SEQ ID No. 7 having an amino acid sequence identity of at least 60 % from the N-terminus in the first 8 AA may be a variant that comprises the sequence: YGZFLRKZ with Z standing for any naturally occurring amino acid and K resubstituting R in position 7, conserving the peptidase recognition site.

Throughout the application "Z" in an amino acid sequence stands for any of the naturally occurring amino acids, in a specific embodiment "Z" may be selected from the group comprising alanine, glycine, asparagine, glutamine, leucine, serine, valine and isoleucine.

According to the present invention said delivery vector comprises a DNA sequence encoding the pre-propeptide of dynorphin or dynorphin-variants. This means said vector comprises a DNA sequence encoding a signal peptide. As one aspect, the present invention provides delivery vectors for transferring a nucleic acid to a cell, the delivery vector comprising a segment encoding a secretory signal peptide. The DNA sequence encoding the signal peptide may be a sequence according to SEQ ID No.: 4. In another aspect of the invention said delivery vector comprises a DNA sequence encoding a propeptide fragment. In a particular aspect of the invention said propeptide fragment is a sequence according to SEQ ID No.5.

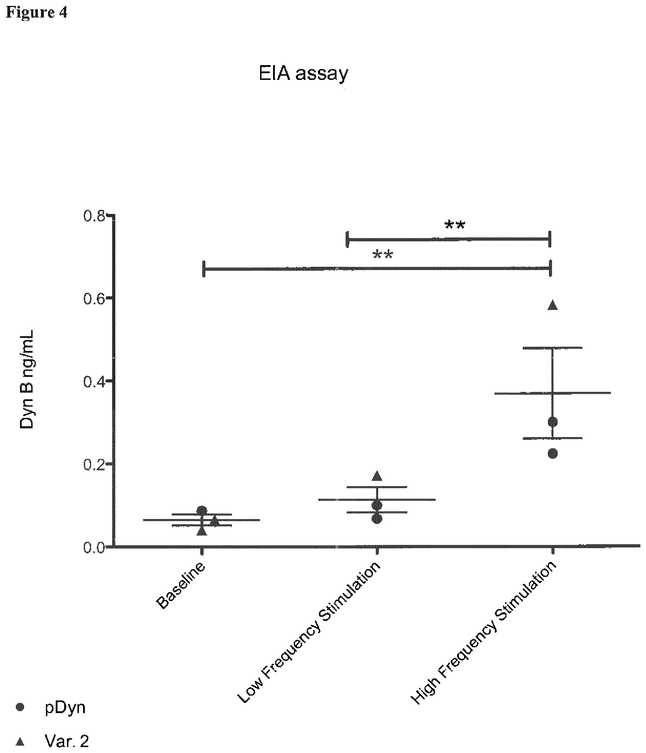

The advantage of the present delivery vectors is a release on demand of dynorphins or dynorphin-variants. This means that prodynorphin (or a variant of prodynorphin) is packed into vesicles, undergoes maturation and is released on demand upon high frequency stimulation (e.g. stimulation ≥ 8 Hz) as it occurs at seizure onset (see Fig 4). A release-on-demand formulation, thus, provides a pre-prodynorphin (or a variant of pre-prodynorphin) that is then packed into vesicles, undergoes maturation and the active substance which is a dynorphin or variant of dynorphin is released upon a frequency of action potentials that exceeds a certain threshold. Said certain threshold maybe a threshold that is ≥ 6 Hz, in another embodiment ≥ 7 Hz in another embodiment ≥ 8 Hz, in another embodiment ≥ 9 Hz. This means said release-on-demand is triggered by increased neuronal firing frequency. Said increased neuronal firing frequency maybe measured by EEG (electroencephalography), increased means a value measured by EEG in said subject that is ≥ 6 Hz, in another embodiment ≥ 7 Hz in another embodiment ≥ 8 Hz, in another embodiment ≥ 9 Hz.

This means that the present delivery vectors drive expression of pre-propeptides that enable the provision of dynorphin or dynorphin-variants on demand as such delivery vectors at first express the pre-propeptides in neurons, where the resulting propeptides are sorted into large dense core vesicles, where it is enzymatically processed and the derived peptides are stored until a sufficiently intense excitation leads to their release, i.e. said release is triggered by increased neuronal firing frequency as explained above. Dyn peptides bind to pre- and/or postsynaptic KOR which activate G-proteins, which, beside others, regulate ion channels to dampen further amplification and spread of neuronal excitation. The translation of the signal peptide of ppDyn is the initial step of the sorting of prodynorphin expressed in neurons into "large dense core" vesicles (LDV). Using existing mechanisms in neurons, the prodynorphin is enzymatically processed to mature peptides and transported to axon terminals. LDV are stored in the axon terminals and released in a stimulation-dependent manner. High frequency stimulation as explained above, like at the onset of seizures, induce the release, while low-frequency stimulation does not. This creates a drug on demand situation. Released Dyn peptides bind to pre- and/or postsynaptic KOR, which activate G-proteins, that, beside others, regulate ion channels to dampen further amplification and spread of neuronal excitation.

In other words a release-on-demand composition is a composition that releases the peptide having agonistic effects on human KOR derived from any of the delivery vectors or recombinant virus particles or liposomes according to the present invention at the onset of seizure in said subject. The onset of seizure may be characterized by increased neuronal firing frequency that maybe measured by EEG (electroencephalography), wherein increased means a value measured by EEG in said subject that is ≥ 6 Hz, in another embodiment ≥ 7 Hz in another embodiment ≥ 8 Hz, in another embodiment ≥ 9 Hz.

A variant of SEQ ID No. 7 having an amino acid sequence identity of at least 60 % from the N-terminus in the first 8 AA is a variant that has an amino acid sequence identity of at least 60 % in the sequence of: YGGFLRRI. Sequence identity is defined as follows: 3 of the first 8 amino acids may be removed or replaced by another amino acid. In any case a variant of SEQ ID No. 7 having an amino acid sequence identity of at least 60 % from the N-terminus in the first 8 AA may be a variant that comprises the sequence: YG-FLRKZ, where "-" stands for a deleted AA, while Z stands for any AA.

A variant of SEQ ID No. 8 having an amino acid sequence identity of at least 60 % from the N-terminus in the first 8 AA is a variant that has an amino acid sequence identity of at least 60 % in the sequence of YGGFLRRQ Sequence identity is defined as follows: 3 of the first 8 amino acids may be removed or replaced by another amino acid. In any case a variant of SEQ ID No. 8 having an amino acid sequence identity of at least 60 % from the N-terminus in the first 8 AA may be a variant that comprises the sequence: YGGFLZZZ, with Z standing for any naturally occurring amino acid.

A variant of SEQ ID No. 9 having an amino acid sequence identity of at least 60 % from the N-terminus in the first 8 AA is a variant that has an amino acid sequence identity of at least 60 % in the sequence of YGGFLRRQ. Sequence identity is defined as follows: 3 of the first 8 amino acids may be removed or replaced by another amino acid. In any case a variant of SEQ ID No. 9 having an amino acid sequence identity of at least 60 % from the N-terminus in the first 8 AA may be a variant that comprises the sequence: YGAFLRZA, with Z standing for any naturally occurring amino acid.

In a specific embodiment subject of the invention is a delivery vector as above described, wherein the variants have an amino acid sequence identity of at least 70 % from the N-terminus in the first 8 AA of SEQ ID No. 7, SEQ ID No. 8 or SEQ ID No. 9, respectively. 70 % sequence identity means that up to 2 of the first 8 amino acids may be removed or replaced by another amino acid.

In a specific embodiment subject of the invention is a delivery vector as above described, wherein the variants have an amino acid sequence identity of at least 80 % from the N-terminus in the first 8 AA of SEQ ID No. 7, SEQ ID No. 8 or SEQ ID No. 9, respectively. 80 % sequence identity means that 1 of the first 8 amino acids may be removed or replaced by another amino acid.

In a specific embodiment subject of the invention is a delivery vector as above described, wherein said delivery vector comprises a DNA sequence encoding a pre-prodynorphin-variant that comprises at least one of the following sequences of variants selected from the group:
SEQ ID No. 10, SEQ ID No. 11 and SEQ ID No. 12 wherein Z may be any naturally occurring amino acid and wherein preferably in SEQ ID No. 10 at least one Z is different in comparison to the sequence SEQ ID No. 7, and wherein preferably in SEQ ID No. 11 at least one Z is different in comparison to the sequence SEQ ID No. 8, and wherein preferably in SEQ ID No. 12 at least one Z is different in comparison to the sequence SEQ ID No. 9.

In a specific embodiment subject of the invention is a delivery vector as above described, wherein said delivery vector comprises multiple DNA sequences encoding SEQ ID No. 7, SEQ ID No. 8 and/or SEQ ID No. 9 or variants thereof wherein the sequences according to SEQ ID No. 7, SEQ ID No. 8 and/or SEQ ID No. 9 or variants thereof are flanked by peptidase recognition signals.

This means as an example that said delivery vector may comprise a DNA sequence encoding SEQ ID No. 7 two times in a way that two molecules of a peptide according to SEQ ID No.7 would be derived from one delivery vector.

Peptidase (prohormone convertase) recognition signals are known to a person skilled in the art and may be single or paired basic amino acids, preferably but not exclusively K, R, KR, RK or RR.

In a specific embodiment subject of the invention is a delivery vector as above described, wherein said delivery vector comprises multiple DNA sequences encoding SEQ ID No. 8 and/or SEQ ID No. 9 or variants thereof wherein the sequences according, SEQ ID No. 8 and/or SEQ ID No. 9 or variants thereof are flanked by peptidase recognition signals.

In a specific embodiment subject of the invention is a delivery vector as above described, wherein said delivery vector comprises multiple DNA sequences encoding SEQ ID No. 8 and/or SEQ ID No. 9 or variants thereof and wherein said delivery vector does not comprise DNA encoding SEQ ID No. 6 and/or 7.

In a specific embodiment subject of the invention is a delivery vector as above described, wherein said delivery vector comprises a DNA encoding SEQ ID No. 2 or SEQ ID No. 14.

In a specific embodiment subject of the invention is a delivery vector as above described, wherein said delivery vector comprises a DNA encoding SEQ ID No. 3 or SEQ ID No. 15.

The delivery vectors produced according to the present invention are useful for the delivery of nucleic acids to cells in vitro, ex vivo, and in vivo. In particular, the delivery vectors can be advantageously employed to deliver or transfer nucleic acids to animal, more preferably mammalian, cells.

Suitable vectors include viral vectors (e.g., retrovirus, lentivirus, alphavirus; vaccinia virus; adenovirus, adeno-associated virus, or herpes simplex virus), lipid vectors, polylysine vectors, synthetic polyamino polymer vectors that are used with nucleic acid molecules, such as plasmids, and the like.

Any viral vector that is known in the art can be used in the present invention. Examples of such viral vectors include, but are not limited to vectors derived from: Adenoviridae; Birnaviridae; Bunyaviridae; Caliciviridae, Capillovirus group; Carlavirus group; Carmovirus virus group; Group Caulimovirus; Closterovirus Group; Commelina yellow mottle virus group; Comovirus virus group; Coronaviridae; PM2 phage group; Corcicoviridae; Group Cryptic virus; group Cryptovirus; Cucumovirus virus group Family ([PHgr]6 phage group; Cysioviridae; Group Carnation ringspot; Dianthovirus virus group; Group Broad bean wilt; Fabavirus virus group; Filoviridae; Flaviviridae; Furovirus group; Group Germinivirus; Group Giardiavirus; Hepadnaviridae; Herpesviridae; Hordeivirus virus group; Illarvirus virus group; Inoviridae; Iridoviridae; Leviviridae; Lipothrixviridae; Luteovirus group; Marafivirus virus group; Maize chlorotic dwarf virus group; icroviridae; Myoviridae; Necrovirus group; Nepovirus virus group; Nodaviridae; Orthomyxoviridae; Papovaviridae; Paramyxoviridae; Parsnip yellow fleck virus group; Partitiviridae; Parvoviridae; Pea enation mosaic virus group; Phycodnaviridae; Picomaviridae; Plasmaviridae; Prodoviridae; Polydnaviridae; Potexvirus group; Potyvirus; Poxviridae; Reoviridae; Retroviridae; Rhabdoviridae; Group Rhizidiovirus; Siphoviridae; Sobemovirus group; SSV 1-Type Phages; Tectiviridae; Tenuivirus; Tetraviridae; Group Tobamovirus; Group Tobravirus; Togaviridae; Group Tombusvirus; Group Tobovirus; Totiviridae; Group Tymovirus; and Plant virus satellites. Protocols for producing recombinant viral vectors and for using viral vectors for nucleic acid delivery can be found in (Ausubel et al., 1989) and other standard laboratory manuals (e.g., Rosenzweig et al. 2007). Particular examples of viral vectors are those previously employed for the delivery of nucleic acids including, for example, retrovirus, lentivirus, adenovirus, adeno-associated virus (AAV) and other parvoviruses, herpes virus, and poxvirus vectors. The term "parvovirus" as used herein encompasses the family Parvoviridae, including autonomous parvoviruses, densoviruses and dependoviruses. The term AAV includes all vertebrate variants especially of human, primate, other mammalian, avian or serpentine origin. The autonomous parvoviruses include members of the genera Parvovirus, Erythrovirus, Bocavirus, Densovirus, Iteravirus, and Contravirus. Exemplary autonomous parvoviruses include, but are not limited to, minute virus of mice, bovine parvovirus, canine parvovirus, chicken parvovirus, feline panleukopenia virus, feline parvovirus, goose parvovirus, HI parvovirus, muscovy duck parvovirus, bocavirus, bufavirus, tusavirus and B19 virus, and any other virus classified by the International Committee on Taxonomy of Viruses (ICTV) as a parvovirus. Other autonomous parvoviruses are known to those skilled in the art. See, e.g. (Berns et al. 2013).

In one embodiment of the invention said delivery vector comprises in addition a recombinant adeno-associated virus (AAV) vector genome or a recombinant lentivirus genome.

In one particular embodiment of the invention said delivery vector comprises in addition a recombinant AAV vector, wherein preferably said vector is a serotype of human or primate origin, in other words said vector is a human or primate serotype vector.

In one particular embodiment of the invention said delivery vector comprises in addition a recombinant adeno-associated virus (AAV) vector genome, wherein said vector is a human serotype vector selected from the group comprising serotypes 1, 2, 3, 4, 5, 6, 7, 8, 9, 10, rh10, 11, 12, 13, 14, serpentine AAV, ancestral AAV, or AAV capsid mutants derived thereof, preferably but not exclusively of AAV serotype 1 or 2.

In one particular embodiment of the invention said delivery vector is a single stranded AAV vector or a self-complimentary (or dimeric) duplex vector.

In one particular embodiment of the invention said delivery vector is a delivery vector as described above, wherein the DNA sequence encoding pre-prodynorphin or pre-prodynorphin-variants is operatively linked to expression control elements comprising a promoter and/or enhancer that produces sufficient expression of the gene product of interest to obtain a therapeutic effect.

For example, the encoding nucleic acid may be operably associated with expression control elements, such as transcription / translation control signals, origins of replication, polyadenylation signals, and internal ribosome entry sites (IRES), promoters, enhancers, and the like. It will further be appreciated that a variety of promoter / enhancer elements may be used depending on the level and tissue-specific expression desired. The promoter / enhancer may be constitutive or inducible, depending on the pattern of expression desired. The promoter / enhancer may be native or foreign and can be a natural or a synthetic sequence. By foreign, it is intended that the transcriptional initiation region is not found in the wild-type host into which the transcriptional initiation region is introduced. Promoter / enhancer elements that are functional in the target cell or subject to be treated are most preferred. Mammalian promoter / enhancer elements are also preferred. Most preferred are promoter / enhancer elements active in human neurons and not, or to a lesser extend in glial cells. The promoter / enhancer element may express the transgene constitutively or inducibly.

Exemplary constitutive promoters include, but are not limited to a Beta-actin promoter, a cytomegalovirus promoter, a cytomegalovirus-enhancer/chicken beta-actin hybrid promoter, and a Rous sarcoma virus promoter. Inducible expression control elements are generally employed in those applications in which it is desirable to provide regulation over expression of the heterologous nucleic acid sequence(s). Inducible promoters / enhancer elements for gene delivery include neuron-specific, brain-specific, muscle specific (including cardiac, skeletal and / or smooth muscle), liver specific, bone marrow specific, pancreatic specific, spleen specific, and lung specific promoter/enhancer elements. In particular embodiments, the promoter/enhancer is functional in cells or tissue of the CNS, and may even be specific to cells or tissues of the CNS. Such promoters / enhancers include but are not limited to promoters/enhancers that function in the eye (e.g., retina and cornea), neurons (e.g., the neuron specific enolase, AADC, synapsin, or serotonin receptor promoter), glial cells (e.g., S100 or glutamine synthase promoter), and oligodendrocytes. Other promoters that have been demonstrated to induce transcription in the CNS include, but are not limited to, myelin basic protein (MBP) promoter (Tani et al., 1996), and the prion promoter (Loftus et al., 2002). Preferred is a neuron-specific promoter displaying significantly reduced, preferably no expression in glial cells.

Other inducible promoter / enhancer elements include drug-inducible, hormone-inducible and metal-inducible elements, and other promoters regulated by exogenously supplied compounds, including without limitation, the zinc-inducible metalothionein (MT) promoter; the dexamethasone (Dex)- inducible mouse mammary tumor virus (MMTV) promoter; the T7 polymerase promoter system (see WO 98/10088); the ecdysone-inducible insect promoter (No et al, 1996); the tetracycline-repressible system (Gossen and Bujard, 1992); the tetracycline-inducible system (Gossen et al., 1995); see also (Harvey et al., 1998); the RU486-inducible system (Wang, DeMayo et al., 1997); (Wang, Xu et al., 1997); and the rapamycin-inducible system (Magari et al., 1997).

In a particular embodiment of the invention the promoter and/or enhancer is selected from the group comprising constitutively active promoters e.g. CMV (cytomegalovirus immediate-early gene enhancer/promoter)- or CBA promoter (chicken beta actin promoter and human cytomegalovirus IE gene enhancer), or inducible promoters comprising Gene Switch, tet-operon derived promotor, or neuron-specific promoters derived of e.g. phosphoglycerate kinase (PGK), synapsin-1 (SYN), neuron-specific enolase (NSE), preferably but not exclusively of human origin.

In a particular embodiment of the invention said delivery vector further comprises a posttranscriptional regulatory element, preferably the woodchuck-hepatitis-virus-posttranscriptional-regulatory element. Other possible posttranscriptional regulatory elements are known to a person skilled in the art.

Subject of the present invention is furthermore the delivery vector according to the present invention, which is a recombinant gene therapy vector comprising the foreign, therapeutic coding sequence, which is flanked by genetic elements for its expression and by virus-specific *cis* elements for its replication, genome packaging, genomic integration etc. The said virus genome is encapsidated as virus particle consisting of virus-specific proteins as in the case of AAV. In the case of lentivirus vectors the viral genome and virus-specific proteins, like reverse transcriptase and others are encapsidated into lentivirus capsids. These are enveloped by a lipid bilayer into which virus-specific proteins are embedded. Liposomes comprise the above described nucleotide sequences or entire DNA backbones including all regulatory elements of the gene therapy-, or delivery vector.

Examples of liposomes include DSPC (1,2-distearoyl-sn-glycero-3-phosphocholine, cholesterol, DSPE-PEG2000 (1,2-distearoyl-sn-glycero-3-phosphoethanol- amine-N-[amino(polyethylene glycol)-2000], or DSPE- PEG2000-mal (1,2-distearoyl-sn-glycero-3-phosphoethanol- amine-N-[maleimide(polyethylene glycol)-2000] or variants comprising sphingomyelin / cholesterol and phosphatidic acid.

In one particular embodiment of the invention said delivery vector comprises in addition a recombinant adeno-associated virus (AAV) vector genome and said recombinant AAV (rAAV) vector genome is encapsidated in an AAV capsid.

Adeno-associated viruses (AAV) have been developed as nucleic acid delivery vectors. For a review, see (Muzyczka, 1992). AAV are helper-dependent parvoviruses requiring a helper virus, typically adenovirus or herpesvirus for productive replication. AAV represent a growing family of currently 14 naturally occurring serotypes of human or primate origin. AAVs of other mammalian species, or of avian or insect origin have been described (see Berns et al., 2013). The AAVs have small icosahedral capsids, 18-26 nanometers in diameter and contain a single-stranded DNA genome of 4 - 5 kilobases in length. AAV encapsidates both AAV DNA strands, either the sense or antisense DNA strand is incorporated into one virion. The AAV genome carries two major open reading frames encoding the genes *rep* and *cap.* Rep encodes a family of overlapping, nonstructural, regulatory proteins. In the best-studied AAV prototype strain, AAV2, the mRNAs for Rep78 and Rep68 are transcribed from the AAV p5 promoter (Stutika et al. 2015). Rep78/68 are required for AAV transcription, AAV DNA replication, AAV integration into the host cell genome and its rescue therefrom. Rep52 and Rep40 represent N-terminally truncated versions of Rep78 and Rep68 transcribed from a separate promoter, p19 and are required for encapsidation of the newly synthesized AAV genome into preformed AAV capsids. These are formed by the three *cap* gene-derived proteins, VP1, VP2, and VP3. The *cap* ORF also encodes AAP, an assembly-enhancing protein that does not form part of the capsid. The AAV ORFs are flanked by inverted terminal repeat sequences (ITRs) at either end of the genome. These vary in length between AAV serotypes, in AAV2 these comprise around 145 bp, the first 125 bp thereof are capable of forming Y- or T-shaped duplex structures. The ITRs represent the minimal AAV sequences required *in cis* for DNA replication, packaging, genomic integration and rescue. Only these have to be retained in an AAV vector to ensure DNA replication and packaging of the AAV vector genome. Foreign genes flanked by AAV-ITRs will be replicated and packaged into AAV capsids provided the AAV genes *rep* and *cap* are expressed *in trans* in the chosen packaging cell (Muzyczka, 1992).

AAV are among the few viruses that can persist over months and years in non-dividing cells *in vivo,* including neurons, muscle, liver, heart and others. Wildtype AAV2 has been shown to integrate its genome into the host cell genome in a Rep78/68-dependent manner, with a preference for chromosomal loci with DNA sequence homology to the so-called Rep-binding site which forms part of the AAV-ITRs (Hüser et al., 2014). In contrast, AAV vectors mostly persist as nuclear episomes. Devoid of the AAV genes *rep* and *cap* AAV vectors rarely integrate at all, and if so without genomic preference (Hüser et al., 2014). Nonetheless long-term AAV persistence has been shown in non-dividing, post mitotic cells including neurons which renders AAV vectors ideal for CNS transduction and long-term gene addition therapy of chronic diseases of genetic or acquired origin.

Generally, a recombinant AAV vector (rAAV) genome will only retain the inverted terminal repeat (ITR) sequence(s) so as to maximize the size of the transgene that can be efficiently packaged by the vector. The structural- and non-structural protein-coding sequences may be provided *in trans,* e.g., from a vector, such as a plasmid, by stably integrating the respective genes into a packaging cell, or in a recombinant helper virus such as HSV or baculovirus, as reviewed in (Mietzsch, Grasse et al., 2014). Typically, the rAAV vector genome comprises at least one AAV terminal repeat, more typically two AAV terminal repeats, which generally will be at the 5' and 3' ends of the heterologous nucleotide sequence(s). The AAV ITR may be from any AAV including serotypes 1-14. Since AAV2-derived ITRs can be crosspackaged into virtually any AAV serotype capsids, AAV2 ITRs combined with AAV2 *rep* are mostly employed. The AAV terminal repeats need not maintain the wild-type terminal repeat sequence (e.g., a wild-type sequence may be altered by insertion, deletion, truncation or missense mutations), as long as the terminal repeat mediates the desired functions, e.g., replication, virus packaging, integration, and/or provirus rescue, and the like. The rAAV vector genome is generally between 80% to about 105% of the size of the wild-type genome and comprises an appropriate packaging signal as part of the AAV-ITR. To facilitate packaging into an AAV capsid, the entire vector genome is preferably below 5.2 kb, more preferably up to 4.8kb in size to facilitate packaging of the recombinant genome into the AAV capsid. So-called dimeric or self-complementary AAV vectors (dsAAV) were developed that package double-stranded instead of single-stranded AAV genomes (McCarty et al., 2001). These lead to enhanced AAV gene expression, however at the price of reduced transgene capacity. Only up to 2 kb of foreign genes can be packaged, which is enough for small genes or cDNAs including those for neuropeptides.

Any suitable method known in the art can be used to produce AAV vectors expressing the nucleic acids of this invention. AAV vector stocks can be produced by co-transfection of plasmids for the ITR-flanked AAV vector genome expressing the transgene together with an AAV rep/cap expressing plasmid of the desired serotype and adenovirus-derived helper genes for AAV replication (Grimm et al., 2003; Xiao et al., 1998). AAV vectors can also be produced in packaging cell lines of mammalian or insect origin and/or in combination with recombinant helperviruses, such as adenovirus, herpes simplex virus (HSV), another member of the herpesvirus family, or baculovirus, as reviewed and discussed in (Mietzsch, Grasse et al., 2014).

Another embodiment of the present invention is a delivery vector or recombinant virus particle or liposome according to the present invention for use in delivering a nucleic acid to a cell of the central nervous system, comprising contacting the cell with the delivery vector or recombinant virus particle as described above under conditions sufficient for the DNA sequence encoding pre-prodynorphin or pre-prodynorphin-variants to be introduced into the cell.

The delivery vectors of the present invention provide a means for delivering nucleic acid sequences into cells of the central nervous system, preferably neurons. The delivery vectors may be employed to transfer a nucleotide sequence of interest to a cell in vitro, e.g., to produce a polypeptide in vitro or for ex vivo gene therapy. The vectors are additionally useful for delivering a nucleotide sequence to a subject in need thereof. In this manner, the polypeptide may thus be produced in vivo in the subject. The subject may be in need of the polypeptide because the subject has a deficiency of the polypeptide, or because the production of the polypeptide in the subject may impart some therapeutic effect, for the use in treatment or otherwise, and as explained further below.

In one particular embodiment encompassing delivering a nucleic acid to a cell of the central nervous system the pre-prodynorphin or pre-prodynorphin-variant is produced and released from the cell.

In one particular embodiment encompassing delivering a nucleic acid to a cell of the central nervous system the method comprises contacting the cell with the recombinant virus particle or liposome as described above under conditions sufficient for the DNA sequence encoding pre-prodynorphin or pre-prodynorphin-variants to be introduced into the cell. Conditions sufficient for the DNA sequence encoding pre-prodynorphin or pre-prodynorphin-variants to be introduced into the cell is the contacting of the AAV capsid to host cell surface receptors and coreceptors. AAV1 capsids bind to 2-3 sialic acid linked to N-acetylgalactosamine, followed by 1-4-linked N-acetylglucosamine, whereas AAV2 capsids bind to heparin sulfate proteoglycan particularly 6-O- and N-sulfated heparins on the cell surface (Mietzsch, Broecker et al., 2014). AAV coreceptors include FGFR-1, Integrin aVb5, hepatocyte growth factor receptor (c-met) and a recently identified, universal AAV receptor, AAVR necessary for transduction with AAV1, AAV2 and others irrespective of the presence of specific glycans (Pillay et al., 2016). AAVR directly binds to AAV particles and helps trafficking to the trans Golgi network. How AAV enters the nucleus is only insufficiently understood. In any case AAV vectors are expressed in the cell nucleus.

One embodiment of the invention is a delivery vector or recombinant virus particle or liposome as described above for use as medicament.

One embodiment of the invention is a delivery vector or recombinant virus particle or liposome as described above for use the preparation of a medicament.

One embodiment of the invention is a delivery vector or recombinant virus particle or liposome according to the present invention for use in treating a diseased subject in need of therapy by administering a delivery vector or recombinant virus particle or liposome as described above.

One embodiment of the invention is a delivery vector or recombinant virus particle or liposome as described above for use of treating focal epilepsy in a subject, in particular mesial temporal lobe epilepsy. One embodiment of the invention is a delivery vector or recombinant virus particle or liposome as described above for use in the preparation of a medicament for treating focal epilepsy in a subject, in particular mesial temporal lobe epilepsy. One embodiment of the invention is a delivery vector or recombinant virus particle or liposome as described above for use in preventing epileptic seizures in a subject that suffers from focal epilepsy whereby said delivery vector or recombinant virus particle or liposome provides activation of human Kappa Opioid Receptors in the epileptogenic focus, thereby inhibiting seizures. In particular said delivery vector or recombinant virus particle or liposome leads to on-demand release of peptides with agonistic effects on human Kappa Opioid Receptors in the epileptogenic focus. Said peptide(s) with agonistic effects on human Kappa Opioid Receptors are in one embodiment selected from the group of peptides having the sequence SEQ ID No. 10, SEQ ID No. 11, SEQ ID No. 12, SEQ ID No. 13, SEQ ID No. 14 or SEQ ID No. 15.

One embodiment of the invention is a delivery vector or recombinant virus particle or liposome according to the present invention for use in treating a diseased subject in need of therapy by administering a delivery vector or recombinant virus particle or liposome as described above wherein said disease is focal, in particular mesial temporal lobe epilepsy.

One embodiment of the invention is a delivery vector or recombinant virus particle or liposome as described above for use in treating focal epilepsy or preventing epileptic seizures in a subject that suffers from focal epilepsy, in particular mesial temporal lobe epilepsy, in a subject, wherein said vector is suitable for transduction of neuronal cells of the central nervous system, e.g. brain.

One embodiment of the invention is a delivery vector or recombinant virus particle or liposome as described above for preparation of a medicament for treating focal epilepsy, in particular mesial temporal lobe epilepsy, in a subject, wherein said vector is suitable for transduction of neuronal cells of the brain.

A vector is suitable for transduction of neuronal cells of the brain means that it attaches to neuronal cell surface receptors and penetrates the cell membrane e.g. by endocytosis.

In a particular embodiment said vector or recombinant virus particle is suitable for peripheral administration or for intracranial or for intracerebral or for intrathecal administration. This is achieved by the aid of a catheter, injection needle or the like, guided by deep brain electrodes to detect the epileptogenic focus.

One embodiment of the invention is a pharmaceutical composition comprising a delivery vector or recombinant virus particle as described above and optionally a pharmaceutically acceptable carrier. Pharmaceutically acceptable carriers are known to a person skilled in the art. Pharmaceutically acceptable carrier may be nanoparticles, liposomes, nanogels, implants releasing vector particles or vector-producing cells.

It is possible to infect a cell [in vitro or ex vivo] with a delivery vector or recombinant virus or liposome particle as described above. For instance the delivery vector or recombinant virus or liposome particle as described above may infect (stem) cells ex vivo during preparation of primary cell culture, or (stem) cells kept in culture. Both, primary cells or stem cells may be derived of the patient to be treated or stem from another subject, or an animal species. Infected cells can be transplanted into the focus of the diseased tissue in order to produce the therapeutic peptides.

Subject of the present invention is a delivery vector or recombinant virus particle or liposome according to the present invention for use in treating a subject with focal epilepsy comprising administering a delivery vector, a recombinant virus particle, or liposome or a pharmaceutical composition as described above to the subject in need thereof.

In an example not, forming part of the literal wording of the claims as granted, the peptides include peptides with agonistic effects on human Kappa Opioid Receptors (KOR) derived or derivable from any of the delivery vectors as described above.

Preferably, said peptides with agonistic effects on KOR are displaying a pKi of 7 or higher for human KOR, measured as described by (Toll et al., 1998).

Preferably, said KOR agonistic peptides displaying pKi of 7 or higher for human KOR exhibit lower pKis for human MU Opioid Receptor (MOR) and human Delta Opioid Receptor (DOR), measured as described by (Toll et al., 1998) Lower pKis for human MOR and human DOR means pKi of 6 or less.

Preferably, said KOR agonistic peptides are displaying pKi of 7.5 or higher for human KOR with lower pKis for human MOR and human DOR, measured as described by (Toll et al., 1998) Lower pKis for human MOR and human DOR means pKi of 6 or less.

In a particular embodiment peptides with agonistic effects on human Kappa Opioid Receptors (KOR) are selected from the group comprising peptides of SEQ ID No. 6, SEQ ID No. 7, SEQ ID No. 8, SEQ ID No. 9, SEQ ID No. 10, SEQ ID No. 11, and SEQ ID No. 12.

### Description of the below sequences:

**SEQ ID No. 1 (ppDyn)** Human pre-prodynorphin before processing as expressed in the human brain.
**SEQ ID No. 2 Variant 1 of ppDyn** 
MAWQGLVLAA CLLMFPSTTA DCLSRCSLCA VKTQDGPKPI NPLICSLQCQ
AALLPSEEWE RCQSFLSFFT PSTLGLNDKE DLGSKSVGEG PYSELAKLSG
SFLKELEKSK FLPSISTKEN TLSKSLEEKL RGLSDGFREG AESELMRDAQ
LNDGAMETGT LYLAEEDPKE QVKRYGGFLR RQFKVVTRSQ EDPNAYSGEL
FDAKRSSEVA GEGDGDSMGH EDLYKRYGGF LRRIRPKLKW DNQKRYGG
FLRRQFKVVT RSQEDPNAYS GELFDA
 Human pre-prodynorphin as in SEQ ID No. 1 engineered so that the peptide neo-endorphin is removed and replaced by a second copy of leumorphin.
**SEQ ID No. 3 Variant 2 of ppDyn** 
MAWQGLVLAA CLLMFPSTTA DCLSRCSLCA VKTQDGPKPI NPLICSLQCQ
AALLPSEEWE RCQSFLSFFT PSTLGLNDKE DLGSKSVGEG PYSELAKLSG
SFLKELEKSK FLPSISTKEN TLSKSLEEKL RGLSDGFREG AESELMRDAQ
LNDGAMETGT LYLAEEDPKE QVKRYGGFLR RQFKVVTRSQ EDPNAYSGEL
FDAKRSSEVA GEGDGDSMGH EDLYKRYGGF LRRQFKVVTR SQEDPNAYSG
ELFDAKRYGG FLRRQFKVVT RSQEDPNAYS GELFDA
 Human pre-prodynorphin variant as in SEQ ID No. 2 engineered so that the peptide Dyn A is removed and replaced by a third copy of leumorphin.
**SEQ ID No. 4 signalpeptide of ppDyn**
   MAWQGLVLAA CLLMFPSTTA
**SEQ ID No. 5 propeptide fragment of ppDyn without known function** 
DCLSRCSLCA VKTQDGPKPI NPLICSLQCQ AALLPSEEWE RCQSFLSFFT
PSTLGLNDKE DLGSKSVGEG PYSELAKLSG SFLKELEKSK FLPSISTKEN
TLSKSLEEKL RGLSDGFREG AESELMRDAQ LNDGAMETGT LYLAEEDPKE QV
**SEQ ID No. 6 Neoendorphin**
   YGGFLRKYP
**SEQ ID No. 7 Dyn A**
   YGGFLRRIRPKLKWDNQ
**SEQ ID No. 8 Dyn B (rimorphin)**
   YGGFLRRQFKVVT
**SEQ ID No. 9: Leumorphin**
   YGGFLRRQFKVVTRSQEDPNAYSGELFDA
**SEQ ID No. 10 Dyn A modified**
   YGZFLRRZRPKLKWDNQ
   Z stands for any naturally occurring amino acid, at least one is preferably substituted by another amino acid in comparison to the wild-type sequence.
**SEQ ID No. 11 Dyn B modified**
   YGZFLRRZFKVVT
   Z stands for any naturally occurring amino acid, at least one is preferably substituted by another amino acid in comparison to the wild-type sequence.
**SEQ ID No. 12: Leumorphin modified**
   YGZFLRRZFKVVTRSQEDPNAYSGELFDA
   Z stands for any naturally occurring amino acid, at least one is preferably substituted by another amino acid in comparison to the wild-type sequence.
**SEQ ID No. 13 (ppDyn modified)** 
MAWQGLVLAA CLLMFPSTTA DCLSRCSLCA VKTQDGPKPI NPLICSLQCQ
AALLPSEEWE RCQSFLSFFT PSTLGLNDKE DLGSKSVGEG PYSELAKLSG
 
SFLKELEKSK FLPSISTKEN TLSKSLEEKL RGLSDGFREG AESELMRDAQ
LNDGAMETGT LYLAEEDPKE QVKRYGGFLR KYPKRSSEVA GEGDGDSMGH
EDLYKRYGZF LRRZRZKLKW DNQKRYGZ FLRRZFKVVT RSQEDPNAYS GELFDA
 Z stands for any naturally occurring amino acid, at least one is preferably substituted by another amino acid in comparison to the wild-type sequence.
**SEQ Id No. 14 (Variant 1 of ppDyn modified)** 
MAWQGLVLAA CLLMFPSTTA DCLSRCSLCA VKTQDGPKPI NPLICSLQCQ
AALLPSEEWE RCQSFLSFFT PSTLGLNDKE DLGSKSVGEG PYSELAKLSG
SFLKELEKSK FLPSISTKEN TLSKSLEEKL RGLSDGFREG AESELMRDAQ
LNDGAMETGT LYLAEEDPKE QVKRYGZFLR RZFKVVTRSQ EDPNAYSGEL
FDAKRSSEVA GEGDGDSMGH EDLYKRYGZF LRRZRZKLKW DNQKRYGZ
FLRRZFKVVT RSQEDPNAYS GELFDA
 Z stands for any naturally occurring amino acid, at least one is preferably substituted by another amino acid in comparison to the wild-type sequence.
**SEQ ID No. 15 Variant 2 of ppDyn modified** 
MAWQGLVLAA CLLMFPSTTA DCLSRCSLCA VKTQDGPKPI NPLICSLQCQ
AALLPSEEWE RCQSFLSFFT PSTLGLNDKE DLGSKSVGEG PYSELAKLSG
SFLKELEKSK FLPSISTKEN TLSKSLEEKL RGLSDGFREG AESELMRDAQ
LNDGAMETGT LYLAEEDPKE QVKRYGZFLR RZFKVVTRSQ EDPNAYSGEL
FDAKRSSEVA GEGDGDSMGH EDLYKRYGZF LRRZFKVVTR SQEDPNAYSG
ELFDAKRYGZ FLRRZFKVVT RSQEDPNAYS GELFDA
 Z stands for any naturally occurring amino acid, at least one is preferably substituted by another amino acid in comparison to the wild-type sequence.

A particular embodiment relates to a delivery vector of the invention, wherein the DNA sequence encoding pre-prodynorphin or pre-prodynorphin-variants is operatively linked to expression control elements comprising a promoter and/or enhancer that produces sufficient expression of the gene product of interest to obtain a therapeutic effect, wherein the promoter and/or enhancer is selected from the group comprising constitutively active promoters e.g. CMV- or CBA promoter (chicken beta actin promoter and human cytomegalovirus IE gene enhancer), or inducible promoters comprising Gene Switch, tet-operon derived promotor, or neuron-specific promoters derived of e.g. phosphoglycerate kinase (PGK), synapsin-1 promoter (SYN), Neuron Specific Enolase (NSE).

Particular embodiments relate to a recombinant virus particle or a liposome, comprising a delivery vector according to the invention.

Particular embodiments relate to the recombinant virus particle or liposome of the invention, wherein said delivery vector comprises in addition a recombinant adeno-associated virus (AAV) vector genome and said rAAV vector genome is encapsidated in an AAV capsid or wherein said delivery vector comprises in addition a recombinant lentivirus vector genome and is packaged in a lentivirus particle.
1. Particular embodiments relate to a delivery vector or recombinant virus particle or liposome for use in delivering a nucleic acid to a cell of the central nervous system according to the invention, comprising contacting the cell with the recombinant virus particle or liposome under conditions sufficient for the DNA sequence encoding pre-prodynorphin or pre-prodynorphin-variants to be introduced into the cell, wherein the pre-prodynorphin or pre-prodynorphin-variant is produced and released from the cell.

Particular embodiments relate to a delivery vector or recombinant virus particle or a liposome according to the invention for use in treating focal epilepsy in a subject, wherein said vector or recombinant virus particle is suitable for peripheral administration or for intracranial or for intracerebral or for intrathecal administration.

Particular embodiments relate to a delivery vector, a recombinant virus particle, or a pharmaceutical composition as defined in embodiments 1 to 17 and 26 for use in treating a subject with focal epilepsy subject.

Particular embodiments relate to a delivery vector according to the invention and wherein said delivery vector drives expression of a pre-propeptide that is pre-prodynorphin or a pre-prodynorphin-variant wherein said pre-propeptide comprise a signalpeptide and, wherein said delivery vector comprises a DNA sequence encoding a pre-prodynorphin-variant that comprises at least one of the following sequences of variants selected from the group:
a. SEQ ID No. 10 (YGZFLRRZRPKLKWDNQ)
b. SEQ ID No. 11 (YGZFLRRZFKVVT)
c. SEQ Id No. 12 (YGZFLRRZFKVVTRSQEDPNAYSGELFDA),
wherein Z stands for any naturally occurring amino acid, and wherein at least one Z in a sequence according to a.; b. or c. is preferably substituted by another amino acid when compared to the wild-type sequence of said dynorphin fragment according to a sequence according to a.; b. or c.

Particular embodiments relate to a delivery vector or recombinant virus particle or a liposome according to the invention for use in treating focal epilepsy in a subject, in particular mesial temporal lobe epilepsy, or for use in preventing epileptic seizures in a subject that suffers from focal epilepsy according to the invention, wherein said vector or recombinant virus particle is suitable for peripheral administration or for intracranial or for intracerebral or for intrathecal or for intraparenchymal administration.

Particular embodiments relate to a delivery vector or recombinant virus particle or a liposome according to the invention for use in treating focal epilepsy in a subject, in particular mesial temporal lobe epilepsy, or for use in preventing epileptic seizures in a subject that suffers from focal epilepsy according to the invention, wherein said delivery vector or recombinant virus particle or a liposome is applied intracerebrally, preferred is applied focally.

Particular embodiments relate to a pharmaceutical release-on-demand composition comprising a delivery vector or recombinant virus particle or liposome according to the invention, and optionally a pharmaceutically acceptable carrier.

A Peptide with agonistic effects on human Kappa Opioid Receptors (KOR) can be derived from any of the delivery vectors according to embodiments 1-6, wherein preferably said peptide is selected from the group comprising the peptides having SEQ ID No. 10, SEQ ID No. 11, SEQ ID No. 12, SEQ ID No. 13, SEQ ID No. 14 and SEQ ID No. 15, e.t. a peptide with agonistic effects on human Kappa Opioid Receptors (KOR) wherein said peptide is selected from the group comprising the peptides having SEQ ID No. 10, SEQ ID No. 11, SEQ ID No. 12, SEQ ID No. 13, SEQ ID No. 14 and SEQ ID No. 15.

Also described herein is a pharmaceutical release-on-demand composition comprising a peptide according to the invention.

### Figure Description

**Figure** 1
   Depicted is an AAV vector backbone including all regulatory genetic elements to express human pre-prodynorphin (red) or □GFP (blue).
**Figure 2**
   EEG recordings obtained from the ipsi-lateral hippocampus of a kainic acid injected animal from two days before to seven days after rAAV-pDyn (SEQ ID No. 1) injection.
**Figure 3**
   Frequency of generalized seizures obtained by EEG recordings from the ipsi-lateral hippocampus and motor cortex of kainic acid injected animals from two days before to 4 months after rAAV-pDyn injection. pDyn = SEQ ID No. 1; Var. 1 = SEQ ID No. 2; Var. 2 = SEQ ID No. 3
**Figure 4**
   Amount of processed Dyn B (Dyn B = SEQ ID No. 8) released during different types of stimulation measured by micro-dialysis and subsequent EIA. pDyn knockout mice were injected with rAAV pDyn (pDyn = SEQ ID No. 1; Var. 2 = SEQ ID No. 3) 3 weeks before the experiment. As these mice do not express endogenous pDyn, all Dyn B measured is vector-derived.
**Figure 5** **(A - F)**
   Barnes maze probe tests from different groups of naive or KA injected animals treated with rAAV. For the memory test the maze is divided in quarters. The Q1 is the one containing the target hole, Q2 and Q4 are the quarters surrounding the Q1 and Q3 is the opposite one. Animals (except those depicted in C) were injected with rAAV either expressing □GFP or pDyn Seq ID no. 1. In A and B, data for mice injected with rAAV, but not treated with kainic acid are depicted. Panel C shows entirely untreated animals. Panels D, E and F show the same set of animals treated with rAAV two weeks after kainic acid at time intervals after kainic acid as stated in the panel.
**Figure 6**
   Pentylenetetrazole is an inhibitor of GABA_{A} receptors and induces seizures upon injection into the tail vein. pDyn deficient animals display a reduce seizure threshold compared to wild-type mice. This can be rescued by treatment with Dyn B. Dyn B variants (Dyn B = SEQ ID No. 8, Dyn B G3A = SEQ ID No. 11 wheren Z at position 3 is alanine, Dyn B G3A + Q8A = SEQ ID No. 11 wheren Z at position 3 is alanine and Z at position 8 of SEQ ID No. 11 is alanine, Dyn B Q8A = SEQ ID No. 11 wheren Z at position 8 is alanine) with a replacement of glycin in position 3 by alanin show higher potency in this test, suggesting that a lower concentration of these peptides can elicit anticonvulsant effects. From this, we expect an early onset of the effect of gene therapy and a lower number of vectors needed per patient.

### References

Ausubel, F. M. et al. (eds.), Current Protocols in Molecular Biology, Greene Publishing Associates, Wiley (1989)
Berns K.I. and C.R. Parrish in FIELDS VIROLOGY, eds. D.N. Knipe and P.M. Howley, volume 2, chapter 57 (6th ed., 2013, Wolters-Kluwer, Lippincott Williams & Wilkins Publishers).
Coatsworth JJ. Studies on the clinical effect of marketed antiepileptic drugs. 1971. NINDS Monograph 12
de Lanerolle NC,Williamson A,Meredith C,Kim JH,Tabuteau H,Spencer DD, Brines ML. Dynorphin and the kappa 1 ligand [3h]u69,593 binding in the human epileptogenic hippocampus. 1997. Epilepsy Res 28:189-205.
Engel JJ. Mesial temporal lobe epilepsy: What have we learned? 2001. Neuroscientist 7:340-352.
Gambardella A,Manna I,Labate A,Chifari R,Serra P,La Russa A,LePiane E,Cittadella R,Andreoli V,Sasanelli F,Zappia M,Aguglia U, Quattrone A. Prodynorphin gene promoter polymorphism and temporal lobe epilepsy. 2003. Epilepsia 44:1255-1256.
Gossen M and Bujard H, (1992) Tight control of gene expression in mammalian cells by tetracyclin-responsive promoters. Proc. Natl. Acad. Sci. USA 89:5547-51.
Gossen M, Freundlieb S, Bender G, Müller G, Hillen W and Bujard H, (1995) Transcriptional activation by tetracyclines in mammalian cells. Science 268(5218):1766-9.
Grimm D, Kay MA, Kleinschmidt JA (2003) Helper virus-free, optically controllable, and two-plasmid-based production of adeno-associated virus vectors of serotypes 1 to 6, Mol Ther 7(6) 839-50
Harvey DM, Caskey CT (1998) Inducible control of gene expression: prospects for gene therapy. Curr. Opin. Chem. Biol. 2:512-8.
Henriksen SJ,Chouvet G,McGinty J, Bloom FE. Opioid peptides in the hippocampus: Anatomical and physiological considerations. 1982. Ann N Y Acad Sci 398:207-220.
Hüser D, Gogol-Döring A, Chen W, Heilbronn R (2014) Adeno-associated virus type 2 wild-type and vector-mediated genomic integration profiles in human diploid fibroblasts analyzed by 3rd generation PacBio DNA sequencing. J Virol l88 (19): 11253-11263
Loacker S,Sayyah M,Wittmann W,Herzog H, Schwarzer C. Endogenous dynorphin in epileptogenesis and epilepsy: Anticonvulsant net effect via kappa opioid receptors. 2007. Brain 130:1017-1028.
Loftus SK, Erickson RP, Walkley SU, Bryant MA, Incao A, Heidenreich RA, Pavan WJ (2002), Rescue of neurodegeneration in Niemann-Pick C mice by a prion promoter-driven Npc1 cDNA transgene. Hum. Mol. Genet. 11:3107-14.
Loscher W, Schmidt D. Modern antiepileptic drug development has failed to deliver: Ways out of the current dilemma. 2011. Epilepsia 52:657-678.
Magari SR Rivera VM, Iulicci JD, Gilman M, Cerasoli F Jr, (1997) Pharmacologic control of a humanized gene therapy system implanted into nude mice J. Clin. Invest. 100:2865-72
McCarty DM, Monahan PE, Samulski RJ (2001) Self-complementary recombinant adeno-associated virus (scAAV) vectors promote efficient transduction independently of DNA synthesis, Gene Ther 8(16) 1248-54
McNamara JO. Emerging insights into the genesis of epilepsy. 1999. Nature 399:A15-22.
Mietzsch M, Broecker F, Reinhardt A, Seeberger PH, Heilbronn R (2014) Differential adeno-associated virus serotype-specific interaction patterns with synthetic heparins and other glycans. J Virol 88: 2991-3003
Mietzsch M, Grasse S, Zurawski C, Weger S, Bennett A, Agbandje-McKenna M, Muzyczka N, Zolotukhin S, Heilbronn R (2014) OneBac: Platform for scalable and high-titer production of adeno-associated virus serotype 1-12 vectors for gene therapy. Hum Gene Ther 25: 212-222
Muzyczka (1992) Use of adeno-associated virus as a general transduction vector for mammalian cells. Curr. Topics Microbiol. Immunol. 158:97-129).
No D, Yao TP Evans RM (1996) Ecdysone-inducible gene expression in mammalian cells and transgenic mice Proc. Natl. Acad. Sci. USA 93:3346.-51
Pillay S, Meyer NL, Puschnik AS, Davulcu O, Diep J, Ishikawa Y, JaeLT, Wosen JE, Nagamine CM, Chapman MS, Carette JE (2016) Nature 530 (7588) 108-12.
Pirker S,Gasser E,Czech T,Baumgartner C,Schuh E,Feucht M,Novak K,Zimprich F, Sperk G. Dynamic up-regulation of prodynorphin transcription in temporal lobe epilepsy. 2009. Hippocampus 19:1051-1054.
Rosenzweig A (2007), Vectors for Gene Therapy. In: Current Protocols in Human Genetics. Wiley John and Sons, Inc.: DOI: 10.1002/0471142905.hg1200s52.
Schunk E,Aigner C,Stefanova N,Wenning G,Herzog H, Schwarzer C. Kappa opioid receptor activation blocks progressive neurodegeneration after kainic acid injection. 2011. Hippocampus 21:1010-1020.
Schwarzer C. 30 years of dynorphins--new insights on their functions in neuropsychiatric diseases. 2009. Pharmacol Ther 123:353-370.
Siggins GR,Henriksen SJ,Chavkin C, Gruol D. Opioid peptides and epileptogenesis in the limbic system: Cellular mechanisms. 1986. Adv Neurol 44:501-512.
Simonato M, Romualdi P. Dynorphin and epilepsy. 1996. Prog Neurobiol 50:557-583.
Solbrig MV,Adrian R,Chang DY, Perng GC. Viral risk factor for seizures: Pathobiology of dynorphin in herpes simplex viral (hsv-1) seizures in an animal model. 2006. Neurobiol Dis 23:612-620.
Spencer S, Huh L. Outcomes of epilepsy surgery in adults and children. 2008. Lancet Neurol 7:525-537.
Stogmann E,Zimprich A,Baumgartner C,Aull-Watschinger S,Hollt V, Zimprich F. A functional polymorphism in the prodynorphin gene promotor is associated with temporal lobe epilepsy. 2002. Ann Neurol 51:260-263.
Stutika C, Gogol-Doring A, Botschen L, Mietzsch M, Weger S, Feldkamp M, Chen W, Heilbronn R (2015) A comprehensive RNA-Seq analysis of the adeno-associated virus type 2 transcriptome reveals novel AAV transcripts, splice variants, and derived proteins. J Virol 90(3) 1278-89
Takahashi M,Senda T,Tokuyama S, Kaneto H. Further evidence for the implication of a kappa-opioid receptor mechanism in the production of psychological stress-induced analgesia. 1990. Jpn J Pharmacol 53:487-494.
Tani M, Fuentes ME, Petersen JW, Trapp BD, Durham SK, Loy JK, Bravo R, Ransohoff RM, Lira SA (1996) Neutrophil infiltration, glial reaction, and neurological disease in transgenic mice expressing the chemokine N51/KC in oligodendrocytes. J. Clin. Invest. 98:529-39.
Toll, L., Berzetei-Gurske, I. P., Polgar, W. E., Brandt, S. R., Adapa, I. D., Rodriguez, L., et al. (1998). Standard binding and functional assays related to medications development division testing for potential cocaine and opiate narcotic treatment medications. NIDA Res Monogr 178, 440-466.
Tortella FC. Endogenous opioid peptides and epilepsy: Quieting the seizing brain? 1988. Trends Pharmacol Sci 9:366-372.
Wang Y, DeMayo FJ, Tsai SY, O'Malley BW (1997) Ligand-inducible and liver-specific target gene expression in transgenic mice. Nat. Biotech. 15:239-43
Wang Y, Xu J, Pierson T, O'Malley BW, Tsai SY (1997) Positive and negative regulation of gene expression in eucaryotic cells with an inducible transcriptional regulator. Gene Ther, 4:432-41.
Xiao X, Li J, Samulski RJ (1998) Production of high-titer recombinant adeno-associated virus vectors in the absence of helper adenovirus, JVirol 72(3) 2224-32
Zangrandi L, Burtscher J, MacKay J, Colmers W, & *Schwarzer C* (2016) The G-protein biased partial kappa opioid receptor agonist 6'-GNTI blocks hippocampal paroxysmal discharges without inducing aversion. British J Pharmacol, 173(11):1756-67,doi: 10.1111/bph.13475

### Examples

### Example 1

### 1.1 AAV constructs

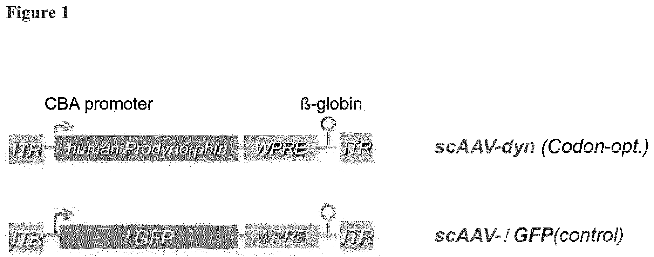

Plasmids with the AAV2-ITR-flanked vector backbones contained the human CMV-IE gene enhancer followed by a truncated version of the chicken beta actin promoter. These drive expression of the full-length codon-optimized human ppdyn sequence (AA SEQ ID No. 1) or either of the variants (AA SEQ ID No. 2; SEQ ID No. 3) or a truncated variant (□GFP) or the full length form of EGFP. Gene expression was enhanced by the woodchuck hepatitis virus posttranslational enhancer element (WPRE) followed by a poly A⁺ signal sequence derived from bovine growth hormone. The AAV2 ITR was either in its wildtype configuration to yield AAV vectors with ssDNA genomes, or one ITR was truncated so that self-complementary AAV vectors with dsDNA genomes resulted (Fig. 1).

### 1.2 AAV vector preparation

HEK 293 cells were seeded at 25-33% confluency in DMEM with 5% FCS. Cells were transfected 24hr later by calcium phosphate cotransfection. AAV vectors were produced essentially as described elsewhere (Mietzsch, Grasse et al., 2014). In brief: The two-plasmid (pDG) cotransfection protocol using plasmids for AAV rep, cap, Ad5 helper genes, and the plasmid for rAAV expressing ppdyn (SEQ ID No. 1) or variants thereof (SEQ ID No. 2; SEQ ID No. 3) as described above. The medium was replaced 12 hr later by medium with reduced FCS content (2%). Cultures were harvested 72hr after transfection by three freeze-thaw cycles for cell lysis. Crude lysates were digested with 250U/ml benzonase (Merck) at 37°C for 1 hr to degrade input and unpackaged plasmid DNA, centrifuged at 8,000 g for 30 min to pellet the cell debris.

### 1.3 rAAV purification

rAAV vectors were packaged in serotypes 1 or 2 capsids and were purified from benzonase treated, cleared freeze-thaw supernatants by one-step heparin sepharose chromatography or by AVB sepharose affinity chromatography using 1 ml prepacked HiTrap columns on an ÄKTA purifier (GE Healthcare) as follows: Freeze-thaw supernatants were diluted 1:1 in 1· phosphate-buffered saline (PBS) supplemented with 1 mM MgCl2 and 2.5 mM KCl (1· PBSMK) before loading on the column at 0.5 ml/min. The column was washed with 20 ml of 1·PBS-MK at a rate of 1 ml / min. AAV vectors were eluted with 0.1 M sodium acetate and 0.5 M NaCl pH 2.5 at a rate of 1 ml / min and neutralized immediately with 1 / 10 volume of 1 M Tris-HCl pH 10. Purified rAAV preparations were dialyzed against 1xPBS-MK using Slide-A-Lyzer dialysis cassettes (10,000 MWCO; Thermo Scientific) (Mietzsch, Grasse et al., 2014).

### 1.4. Quantification of rAAV vector preparations

Highly purified rAAV vector preparations were digested with Proteinase K for 2 hr at 56°C. DNA was purified by repeated extractions with phenol and chloroform and precipitated with ethanol. Serial dilutions of capsid-released AAV genomes were analyzed by quantitative Light-Cycler PCR, using the Fast Start DNA Master SYBR Green kit (Roche). PCR primers were specific for the bovine growth hormone gene-derived polyA site of the vector backbone (5'-CTAGAGCTCGCTGATCAGCC-3' and 5'-TGTCTTCCCAATCCTCCCCC-3'). The titer of the highly purified AAV preparations was measured as AAV-DNA containing genomic particles (gp) / ml (Mietzsch, Grasse et al., 2014).

### Example 2

### Animals

C57BL/6N wild-type and pDyn knockout (pDyn-KO) mice were investigated in this study. pDyn-KO mice were backcrossed onto the C57BL/6N background over 10 generations (Loacker et al., 2007). For breeding and maintenance, mice were group-housed with free access to food and water. Temperature was fixed at 23°C and 60% humidity with a 12 h light-dark cycle (lights on 7 am to 7 pm). All procedures involving animals were approved by the Austrian Animal Experimentation Ethics Board in compliance with the European Convention for the Protection of Vertebrate Animals Used for Experimental and Other Scientific Purposes ETS no.: 123, and the Canadian Council on Animal Care. Every effort was taken to minimise the number of animals used.

### Example 3

### Kainic acid injection and electrodes implantation

Male mice (12-14 weeks) were sedated with ketamine (160 mg / kg, i.p.; Graeub Veterinary Products, Switzerland) and then deeply anesthetised with sevoflurane through a precise vaporizer (Midmark, USA). Mice were injected with 50 nl of a 20 mM KA solution into the hippocampus (RC -1.80 mm; ML +1.80 mm; DV -1.60 mm) as previously described (Loacker et al., 2007). Two electrodes (one cortical and one depth electrodes) were implanted immediately after KA administration. Epoxylite-coated tungsten depth electrodes (diameter 250 µm; FHC, USA) were placed into the hippocampus aimed at the CA1 area (RC -1.80 mm; ML +1.80 mm; DV -1.60 mm). Surface electrodes were gold-plated screws placed into the skull on top of the motor-cortex (RC +1.70 mm; ML +1.6 mm with the bregma as a reference point) to monitor the generalisation of abnormal EEG activities. An additional surface electrode was placed on the cerebellum as ground and reference. Electrodes were secured in place with dental acrylate (Heraeus Kulzer GmbH, Germany).

### Example 4

### rAAV injections

For experiments requiring rAAV administration, a guide cannula was implanted, which was attached to the hippocampal depth electrode. All animals received meloxicam (2 mg / kg) 20 minutes before surgery as an analgesic treatment. For the rAAVs injections animals were mildly anesthetized in a sevoflurane chamber during the time of the injection (20 minutes). The injection was made through the guide cannula with an injection pump at a flow of 0,1 µL/min, a total volume of 2 µL was injected.

### Example 5

### EEG recoding and analysis

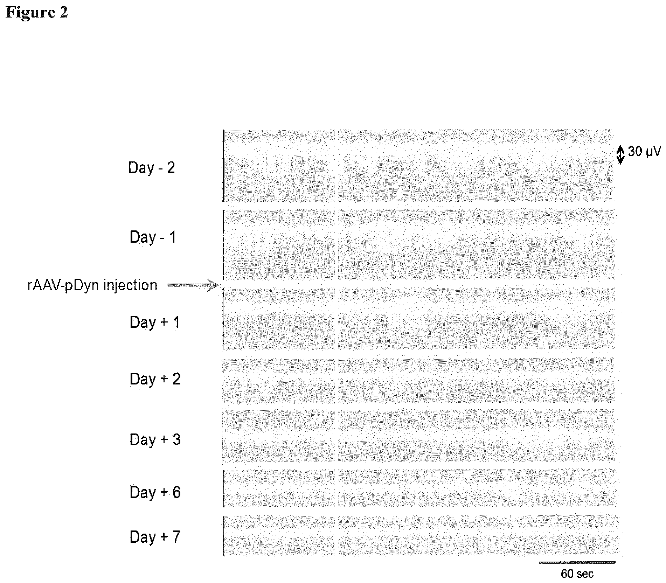

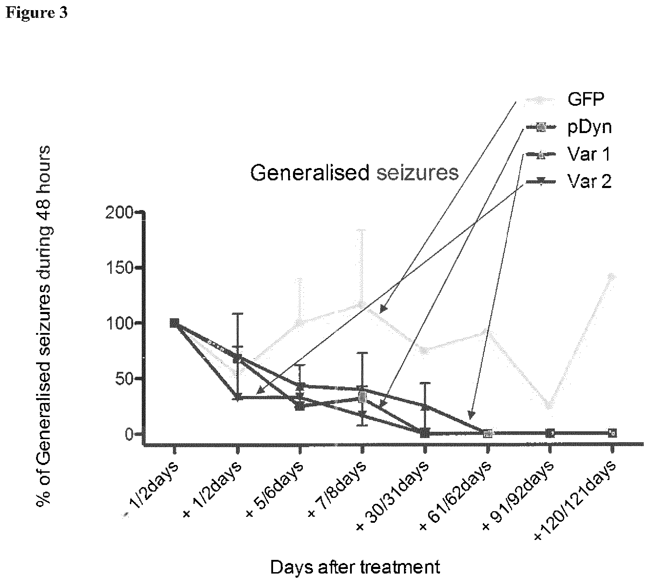

The EEG was obtained using a wireless recording device (Neurologger, TSE, Germany) and automatically analysed using SciWorks Software (Datawave Technologies, USA). EEGs were filtered for epileptiform spikes defined as a high amplitude discharges (> 3 × baseline) lasting less than 70 ms. Spike trains were defined as the occurrence of at least three spikes with a frequency higher than 1 Hz and lasting for at least 1 s (see Fig. 2). Spikes with lower frequencies were counted as inter-ictal spikes. Prolonged hippocampal paroxysmal discharges (hpd) were defined as spike trains lasting for a minimum of 10 sec (Zangrandi et al., 2016). Generalized seizures were assessed visually by co-appearance for high voltage EEG abnormalities in the hippocampal depth and motor-cortical surface electrode (see Fig. 3).

### Example 6

### Spatial memory testing (Barnes maze)

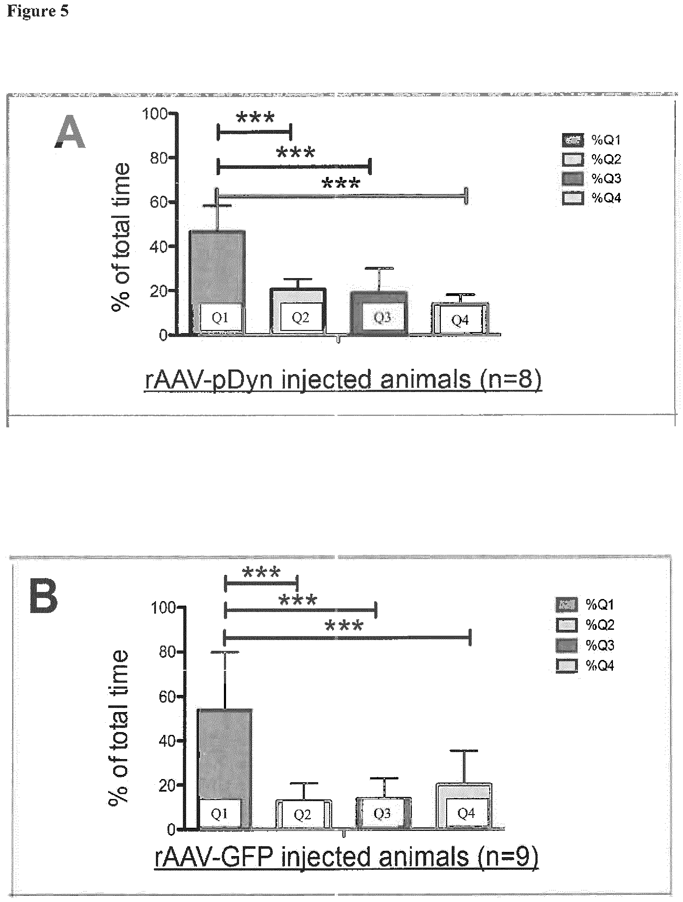

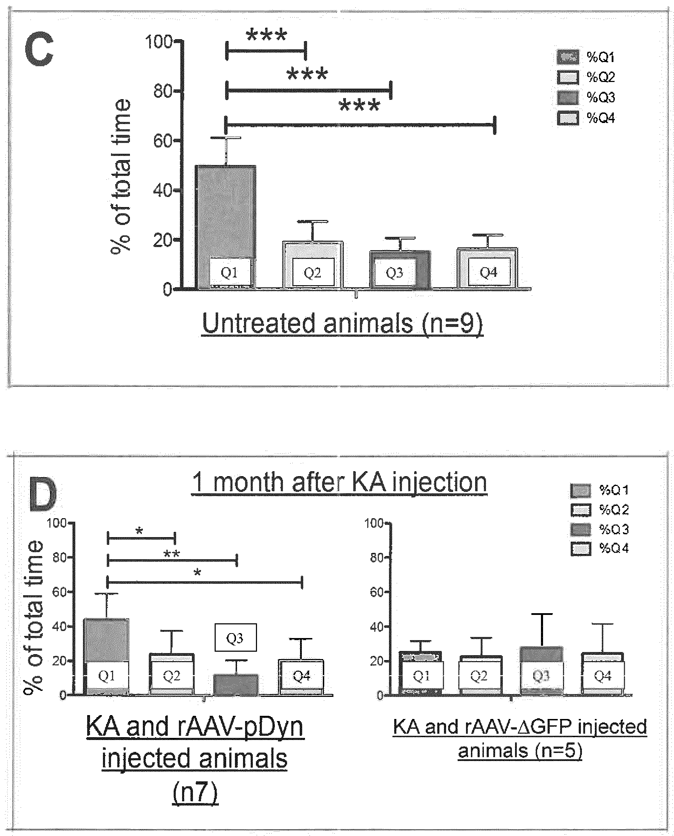

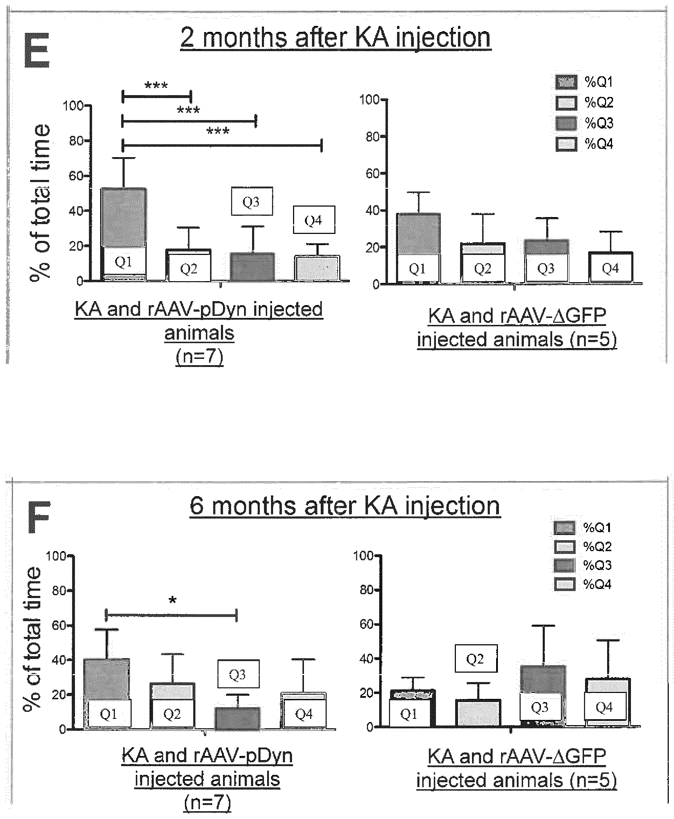

To assess learning and memory of naive and treated animals the Barnes maze was used. the Barnes maze was executed at 60 lux on a flat circular table (diameter 100 cm) with 20 holes around its perimeter. Amongst those, only one allowed the mouse to exit the maze into an escape dark box. Visual clues were placed around the disk with an interval of 90°. The first day was the habituation day, mice were allowed to freely explore the maze during 5 minutes with the target hole open. The position of the escape box was kept constant during the entire experiment. When the mouse found the hole, the box was closed and the animal was kept in there for 2 minutes to let it associate the escape box as a secure place. If the animal didn't find the target hole during the 5 minutes, the animal was gently guided to the hole. Acquisition was made during the next 4 days. 3 trials of 3 minutes maximum were performed, as soon as the mouse find the hole, it was kept for 2 minutes inside. If the mice didn't find it after the 3 minutes it was gently guide to the hole. The primary errors done by the animal and the latency to find the hole were calculated and defined as learning criteria. The probe trial was executed one the sixth day. All the holes were closed and the mouse was free to explore the maze during 5 minutes. For evaluation, the board was divided in quadrants and the time spent in each quadrant was measured (see Fig. 5). The quadrant previously containing the open escape hole is referred to as Q1, this is flanked by Q2 and Q4, while Q3 is opposing Q1.

### Example 7

### Microdialysis

Microdialysis was performed on pDyn-KO animals which had received rAAV-pDyn injection into one hippocampus as described above 3 weeks before the microdialysis experiment. At the time of vector injection (RC -1.80 mm; ML +1.80 mm; DV -1.60 mm), animals were implanted with a stimulation electrode (RC -4.20 mm; ML +3.20 mm; DV -4.90 mm) and a guide cannula targeting the hilus of the rAAV-injected hippocampus. For microdialysis MAB-2 probes (SciPro, Sanborn, NY) were placed into the hippocampus and flushed by artificial CSF (140mM NaCl; 3.0 mM KCl; 1.25 mM CaCl₂; 1.0 mM MgCl₂; 1.2 mM Na₂HPO₄; 0.3 mM NaH₂PO₄; 3mM glucose, pH 7.2) at a rate of 0.4 µL/min. ACSF was collected for 3 x 25 min followed by 25 min low frequency stimulation (300 µA; isolated 0.3 msec square pulses with 10 sec interval, ISO-STIM 01D, NPI, Tamm, Germany). After another 25 min baseline, 25 min of high frequency stimulation were performed (150 µA; trains of 0.3 msec square pulses 20 msec apart for 1 sec; trains were separated by 10 sec.).

### Example 8

### Dynorphin B enzyme immunoassay (EIA)

The content of processed Dynorphin B in the eluate of microdialysis experiments was measured by a specific Dyn B (SEQ ID No. 8) EIA (S-1429; Peninsula; San Carlos, CA), according to manufacturer's manual. In short, samples were incubated with the antiserum for 1 hour, followed by an overnight incubation with the Bt-tracer. On the second day, streptavidin-HRP was added for one hour after five washes with EIA buffer. After another 5 washes, samples were reacted with TMB solution for 5 minutes and then analysed on a plate reader a 450 nm. Dyn B (SEQ ID No. 8) content was analysed based on calibration samples run in parallel and expressed as ng/ml (see Fig. 4).

### Example 9

### Statistical Analysis

Following acquisition, electrophysiological recordings were viewed and analysed using pClamp 10.3 (Molecular devices). Prism 5 for Mac (version 5.0f) was used to perform a statistical analysis of in vivo experiments and to generate figures. For the statistical analysis, a one-way ANOVA with a Dunnett post hoc test was applied to in vivo experiments. For electrophysiology, the two-tailed, paired t-tests were applied for resting membrane potential analysis, and a one-way ANOVA was used to compare drug effects on IPSC. A p value less than 0.05 was considered significant. Data are presented as mean ± standard error of the mean (SEM).

### Example 10

### Seizure threshold

The seizure threshold is a measure for the susceptibility to develop seizures. The resistance against seizure-inducing agents or stimuli is used as readout in animals. Infusion of pentylenetetrazole, a GABA_{A} receptor antagonist, into the tail vein of rodents is an accepted method to measure the seizure threshold. Anticonvulsant activity of substances or treatments can be demonstrated by an increased seizure threshold upon application. pDyn deficient mice display a lower seizure threshold than wild-type mice. This can be rescued by kappa opioid receptors agonists.

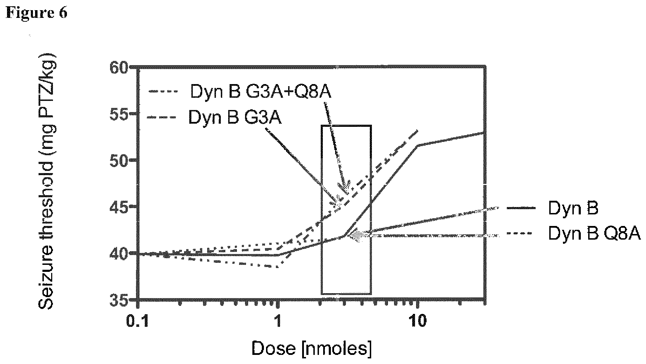

Seizure threshold was determined by pentylenetetrazole (PTZ) (see Fig. 6) tail-vein infusion on freely moving pDyn deficient mice at a rate of 100 µl/min (100 µg/ml PTZ in saline, pH 7.4). Infusion was stopped when animals displayed generalized clonic seizures. The seizure threshold dose was calculated from the infused volume in relation to body weight. Dyn B or modified variants thereof were dissolved in DMSO and diluted to the final dosages in saline containing final concentrations of 10 % DMSO, 3 % Tween 80. 3 µl of peptide-solution were applied 30 min. before tail-vein infusion under mild sevoflurane anaesthesia into the cisterna magna. The following peptides were tested: Dyn B = SEQ ID No. 8, Dyn B G3A = SEQ ID No. 11 wherein Z at position 3 is alanine, Dyn B G3A + Q8A = SEQ ID No. 11 wherein Z at position 3 is alanine and Z at position 8 of SEQ ID No. 11 is alanine, Dyn B Q8A = SEQ ID No. 11 wherein Z at position 8 is alanine.

## Claims

1. A delivery vector comprising a DNA sequence encoding pre-prodynorphin or pre-prodynorphin-variants and wherein said delivery vector drives expression of a pre-propeptide that enables the release of dynorphin or dynorphin-variants on demand, wherein said pre-propeptide is pre-prodynorphin or a pre-prodynorphin-variant and wherein said pre-propeptide comprise a signalpeptide that enables the packing of said pro-peptide into vesicles, and wherein said delivery vector leads to release-on-demand of dynorphins or dynorphin-variants , whereas said pre-prodynorphin or pre-prodynorphin-variants comprise at least one of the following sequences selected from the group:
a. Dyn A that is SEQ ID No. 7 (AA 207-223 of SEQ ID No. 1; ppDyn) or a variant thereof consisting of the first 13 AA (first from the N-terminal end that is YGGFLRRIRPKLK (SEQ ID No. 16)) or a variant thereof consisting of the first 8 AA (first from the N-terminal end that is YGGFLRRI (SEQ ID No. 17))
b. Dyn B that is SEQ ID No. 8 (AA 226-238 of SEQ ID No. 1; ppDyn)
c. leumorphin that is SEQ ID No. 9 (AA 226-254 of SEQ ID No. 1; ppDyn)
d. variants of Dyn A according to SEQ ID No.7 having an amino acid sequence identity of at least 60 % within the first 8 AA counted from the N-terminus of SEQ ID No. 7 (YGGFLRRI (SEQ ID No. 17)) i.e. having an amino acid sequence identity of at least 60 % within the sequence YGGFLRRI comprised in SEQ ID No. 7.
e. variants of Dyn B according to SEQ ID No. 8 having an amino acid sequence identity of at least 60 % within the first 8 AA counted from the N-terminus of SEQ ID No. 8 (YGGFLRRQ (SEQ ID No. 18)) i.e. having an amino acid sequence identity of at least 60 % within the sequence YGGFLRRQ comprised in SEQ ID No. 8.
f. variants of leumorphin according to SEQ ID No. 9 having an amino acid sequence identity of at least 60 % within the first 8 AA counted from the N-terminus of SEQ ID No. 9 (YGGFLRRQ (SEQ ID No. 18)), i.e. having an amino acid sequence identity of at least 60 % within the sequence YGGFLRRQ comprised in SEQ ID No. 9.

2. A delivery vector according to claim 1, wherein said delivery vector leads to release-on-demand of dynorphins or dynorphin-variants with agonistic effects on human Kappa Opioid Receptors.

3. A delivery vector according to claim 1 or claim 2, wherein the variants have an amino acid sequence identity of at least 70 % within the first 8 AA counted from the N-terminus of SEQ ID No. 7 (YGGFLRRI), SEQ ID No. 8 (YGGFLRRQ) or SEQ ID No. 9 (YGGFLRRQ), respectively.

4. A delivery vector according to claims 1 to 3, wherein the variants have an amino acid sequence identity of at least 80 % within the first 8 AA counted from the N-terminus of SEQ ID No. 7, SEQ ID No. 8 or SEQ ID No. 9, respectively.

5. A delivery vector according to any of claims 1 to 4 and wherein said delivery vector drives expression of a pre-propeptide that is pre-prodynorphin or a pre-prodynorphin-variant wherein said pre-propeptide comprises a signalpeptide and, wherein said delivery vector comprises a DNA sequence encoding a pre-prodynorphin-variant that comprises at least one of the following sequences of variants selected from the group:
a. SEQ ID No. 10 (YGZFLRRZRPKLKWDNQ)
b. SEQ ID No. 11 (YGZFLRRZFKVVT)
c. SEQ Id No. 12 (YGZFLRRZFKVVTRSQEDPNAYSGELFDA),
wherein Z stands for any of the naturally occurring amino acid, and wherein at least one Z in a sequence according to a.; b. or c. is preferably substituted by another amino acid when compared to the wild-type sequence of said dynorphin fragment according to a sequence according to a.; b. or c.

6. A delivery vector according to any of claims 1 to 5, and wherein said delivery vector drives expression of a pre-propeptide that is pre-prodynorphin or a pre-prodynorphin-variant wherein said pre-propeptide comprises a signalpeptide and, wherein said delivery vector comprises a DNA sequence encoding a pre-prodynorphin-variant, wherein "Z" is selected from the group comprising alanine, glycine, asparagine, glutamine, leucine, serine, valine and isoleucine.

7. A delivery vector according to any of claims 1 to 6, wherein said delivery vector comprises in addition a recombinant adeno-associated virus (AAV) vector genome or a recombinant lentivirus genome.

8. A delivery vector according to any of claims 1 to 7 comprising a recombinant adeno-associated virus (AAV) vector genome, wherein said vector is a human serotype vector selected from the group comprising serotypes 1, 2, 3, 4, 5, 6, 7, 8, 9, 10, rh10, 11, 12, 13, 14, serpentine AAV, ancestral AAV or AAV capsid mutants derived thereof, preferably of serotype 1 or 2.

9. A delivery vector according to claims 1 to 8 , wherein said delivery vector drives expression of a pre-propeptide that is pre-prodynorphin or a pre-prodynorphin-variant wherein said pre-propeptide comprises a signalpeptide and, wherein said delivery vector comprises a DNA sequence encoding a pre-prodynorphin-variant that comprises at least one of the following sequences of variants selected from the group comprising the peptides having SEQ ID No. 10, SEQ ID No. 11, SEQ ID No. 12, SEQ ID No. 13, SEQ ID No. 14 and SEQ ID No. 15.

10. A recombinant virus particle or a liposome, comprising a delivery vector according to any of the preceding claims.

11. The recombinant virus particle or liposome of claim 10, wherein said delivery vector comprises in addition a recombinant adeno-associated virus (AAV) vector genome and said rAAV vector genome is encapsidated in an AAV capsid or wherein said delivery vector comprises in addition a recombinant lentivirus vector genome and is packaged in a lentivirus particle.

12. A delivery vector or recombinant virus particle or liposome according to any of claims 1 to 11 for use in delivering a nucleic acid to a cell of the central nervous system, comprising contacting the cell with the delivery vector or recombinant virus particle or liposome under conditions sufficient for the DNA sequence encoding pre-prodynorphin or pre-prodynorphin-variants to be introduced into the cell.

13. A delivery vector or recombinant virus particle or liposome according to any of claims 1 to 11 for use as medicament.

14. A delivery vector or recombinant virus particle or liposome according to any of claims 1 to 11 and 13 for use in treating focal epilepsy in a subject, in particular mesial temporal lobe epilepsy, or for use in preventing epileptic seizures in a subject that suffers from focal epilepsy whereby said delivery vector or recombinant virus particle or liposome provides activation of human Kappa Opioid Receptors in the epileptogenic focus, thereby inhibiting seizures.

15. A delivery vector or recombinant virus particle or liposome according to any of claims 1 to 11 and 13 for use in treating focal epilepsy in a subject, in particular mesial temporal lobe epilepsy, or for use in preventing epileptic seizures in a subject that suffers from focal epilepsy whereby said delivery vector or recombinant virus particle or liposome provides activation of human Kappa Opioid Receptors in the epileptogenic focus, thereby inhibiting seizures whereby said delivery vector or recombinant virus particle or liposome leads to release-on-demand of peptides with agonistic effects on human Kappa Opioid Receptors in the epileptogenic focus.

16. A delivery vector or recombinant virus particle or a liposome according to any of claims 1 to 11 and 13 for use in treating focal epilepsy in a subject, in particular mesial temporal lobe epilepsy, or for use in preventing epileptic seizures in a subject that suffers from focal epilepsy according to claims 13-14, wherein said vector or recombinant virus particle is suitable for peripheral administration or for intracranial or for intracerebral or for intrathecal or for intraparenchymal administration.

17. A delivery vector or recombinant virus particle or a liposome according to any of claims 1 to 11 and 13 for use in treating focal epilepsy in a subject, in particular mesial temporal lobe epilepsy, or for use in preventing epileptic seizures in a subject that suffers from focal epilepsy according to claims 13-14, wherein said delivery vector or recombinant virus particle or a liposome is applied intracerebrally, preferred is applied focally.

18. A pharmaceutical release-on-demand composition comprising a delivery vector or recombinant virus particle or liposome according to any of claims 1 to 11 and 13, and optionally a pharmaceutically acceptable carrier.

19. A delivery vector or a recombinant virus particle as defined in claims 1 to 11 and 13, or a pharmaceutical composition according to claim 18 for use in treating focal epilepsy in a subject, in particular mesial temporal lobe epilepsy, or for use in preventing epileptic seizures in a subject that suffers from focal epilepsy,
whereby preferably said delivery vector or recombinant virus particle or liposome encodes pre-propeptides, which after maturation and release provide activation of human Kappa Opioid Receptors in the epileptogenic focus, thereby inhibiting seizures, and wherein preferably said delivery vector or recombinant virus particle or a liposome is applied intracerebrally, preferably applied focally.

## Patentansprüche

1. Verabreichungsvektor umfassend eine DNA-Sequenz, die Pre-Prodynorphin oder Pre-Prodynorphin-Varianten kodiert und wobei der Verabreichungsvektor die Expression eines Pre-Propeptids vermittelt, das auf Anforderung die Freisetzung von Dynorphin oder Dynorphin-Varianten ermöglicht, wobei das Pre-Propeptid Pre-Prodynorphin oder eine Pre-Prodynorphin-Variante ist und wobei das Pre-Propeptid ein Signalpeptid umfasst, das die Verpackung des Pre-Propeptids in Vesikel ermöglicht, und wobei der Verabreichungsvektor zur Freisetzung-auf-Anforderung von Dynorphinen oder Dynorphin-Varianten führt, wobei das Pre-Prodynorphin oder die Pre-Prodynorphin-Varianten mindestens eine der folgenden Sequenzen umfassen, die ausgewählt sind aus der Gruppe:
a. Dyn A, das SEQ-ID-NR. 7 (AA 207-223 von SEQ-ID-NR. 1; ppDyn) oder eine Variante davon ist, bestehend aus den ersten 13 Aminosäuren (gezählt ab dem N-terminalem Ende, das YGGFLRRIRPKLK (SEQ-ID-NR. 16) ist) oder eine Variante davon bestehend aus den ersten 8 Aminosäuren (ab dem N-terminalem Ende, das YGGFLRRI (SEQ-ID-NR. 17) ist)
b. Dyn B, das SEQ-ID-NR. 8 (AA 226-238 von SEQ-ID-NR. 1; ppDyn) ist
c. Leumorphin, das SEQ-ID-NR. 9 (AA 226-254 von SEQ-ID-NR. 1; ppDyn) ist
d. Varianten von Dyn A nach SEQ-ID-NR. 7 mit einer Aminosäuresequenz-Identität von mindestens 60 % innerhalb der ersten 8 Aminosäuren, gezählt ab dem N-Terminus von SEQ-ID-NR. 7 (YGGFLRRI (SEQ-ID-NR. 17)), d. h. mit einer Aminosäuresequenz-Identität von mindestens 60 % innerhalb der Sequenz YGGFLRRI, die von SEQ-ID-NR. 7 umfasst ist.
e. Varianten von Dyn B nach SEQ-ID-NR. 8 mit einer Aminosäuresequenz-Identität von mindestens 60 % innerhalb der ersten 8 Aminosäuren, gezählt ab dem N-Terminus von SEQ-ID-NR. 8 (YGGFLRRQ (SEQ-ID-NR. 18)), d. h. mit einer Aminosäuresequenz-Identität von mindestens 60 % innerhalb der Sequenz YGGFLRRQ, die von SEQ-ID-NR. 8 umfasst ist.
f. Varianten von Leumorphin nach SEQ-ID-NR. 9 mit einer Aminosäuresequenz-Identität von mindestens 60 % innerhalb der ersten 8 Aminosäuren, gezählt ab dem N-Terminus von SEQ-ID-NR. 9 (YGGFLRRQ (SEQ-ID-NR. 18)), d. h. mit einer Aminosäuresequenz-Identität von mindestens 60 % innerhalb der Sequenz YGGFLRRQ, die von SEQ-ID-NR. 9 umfasst ist.

2. Verabreichungsvektor nach Anspruch 1, wobei der Verabreichungsvektor zur Freisetzung-auf-Anforderung von Dynorphinen oder Dynorphin-Varianten mit agonistischen Effekten auf menschliche Kappa-Opioidrezeptoren führt.

3. Verabreichungsvektor nach Anspruch 1 oder 2, wobei die Varianten eine Aminosäuresequenz-Identität von mindestens 70 % innerhalb der ersten 8 Aminosäuren haben, gezählt jeweils ab dem N-Terminus von SEQ-ID-NR. 7 (YGGFLRRI), SEQ-ID-NR. 8 (YGGFLRRQ) oder SEQ-ID-NR. 9 (YGGFLRRQ).

4. Verabreichungsvektor nach Anspruch 1 bis 3, wobei die Varianten eine Aminosäuresequenz-Identität von mindestens 80 % innerhalb der ersten 8 Aminosäuren haben, gezählt jeweils ab dem N-Terminus von SEQ-ID-NR. 7, SEQ-ID-NR. 8 oder SEQ-ID-NR. 9.

5. Verabreichungsvektor nach einem der Ansprüche 1 bis 4 und wobei der Verabreichungsvektor die Expression eines Pre-Propeptids vermittelt, das Pre-Prodynorphin oder eine Pre-Prodynorphin-Variante ist, wobei das Pre-Propeptid ein Signalpeptid umfasst, und wobei der Verabreichungsvektor eine DNA-Sequenz umfasst, die eine Pre-Prodynorphin-Variante kodiert, die mindestens eine der folgenden Sequenzen von Varianten umfasst, die ausgewählt sind aus der Gruppe:
a. SEQ-ID-NR. 10 (YGZFLRRZRPKLKWDNQ)
b. SEQ-ID-NR. 11 (YGZFLRRZFKVVT)
c. SEQ-ID-NR. 12 (YGZFLRRZFKVVTRSQEDPNAYSGELFDA),
wobei Z für jegliche der natürlich vorkommenden Aminosäuren steht, und wobei mindestens ein Z in einer Sequenz nach a.; b. oder c. verglichen mit der Wildtyp-Sequenz des Dynorphin-Fragments nach einer Sequenz nach a.; b. oder c. vorzugsweise durch eine andere Aminosäure substituiert.

6. Verabreichungsvektor nach einem der Ansprüche 1 bis 5 und wobei der Verabreichungsvektor die Expression eines Pre-Propeptids vermittelt, das Pre-Prodynorphin oder eine Pre-Prodynorphin-Variante ist, wobei das Pre-Propeptid ein Signalpeptid umfasst, und wobei der Verabreichungsvektor eine DNA-Sequenz umfasst, die eine Pre-Prodynorphin-Variante kodiert, wobei Z ausgewählt ist aus der Gruppe umfassend Alanin, Glycin, Asparagin, Glutamin, Leucin, Serin, Valin und Isoleucin.

7. Verabreichungsvektor nach einem der Ansprüche 1 bis 6, wobei der Verabreichungsvektor zusätzlich ein rekombinantes Adeno-assoziiertes Virus (AAV)-Vektorgenom oder ein rekombinantes Lentivirusgenom umfasst.

8. Verabreichungsvektor nach einem der Ansprüche 1 bis 7, umfassend ein rekombinantes Adeno-assoziiertes Virus (AAV)-Vektorgenom, wobei der Vektor ein menschlicher Serotypvektor ist, ausgewählt aus der Gruppe umfassend Serotypen 1, 2, 3, 4, 5, 6, 7, 8, 9, 10, rh10, 11, 12, 13, 14, Serpentin AAV, vererbtes AAV oder davon abgeleitete AAV-Kapsidmutanten, vorzugsweise Serotyp 1 oder 2.

9. Verabreichungsvektor nach einem der Ansprüche 1 bis 8, wobei der Verabreichungsvektor die Expression eines Pre-Propeptids vermittelt, das Pre-Prodynorphin oder eine Pre-Prodynorphin-Variante ist, wobei das Pre-Propeptid ein Signalpeptid umfasst, und wobei der Verabreichungsvektor eine DNA-Sequenz umfasst, die eine Pre-Prodynorphin-Variante kodiert, die mindestens eine der folgenden Sequenzen von Varianten umfasst, die ausgewählt sind aus der Gruppe umfassend die Peptide mit SEQ-ID-NR. 10, SEQ-ID-NR. 11, SEQ-ID-NR. 12, SEQ-ID-NR. 13, SEQ-ID-NR. 14 und SEQ-ID-NR. 15.

10. Rekombinantes Viruspartikel oder Liposom, umfassend einen Verabreichungsvektor nach einem der vorhergehenden Ansprüche.

11. Rekombinantes Viruspartikel oder Liposom nach Anspruch 10, wobei der Verabreichungsvektor zusätzlich ein rekombinantes Adeno-assoziiertes Virus (AAV)-Vektorgenom umfasst und das rAAV-Vektorgenom in einem AVV-Kapsid eingehüllt ist oder wobei der Verabreichungsvektor zusätzlich ein rekombinantes Lentivirus-Vektorgenom umfasst und in einem Lentiviruspartikel verpackt ist.

12. Verabreichungsvektor oder rekombinantes Viruspartikel oder Liposom nach einem der Ansprüche 1 bis 11 zur Verwendung beim Transport einer Nukleinsäure zu einer Zelle des zentralen Nervensystems, umfassend das In-Kontakt-Bringen der Zelle mit dem Verabreichungsvektor oder dem rekombinanten Viruspartikel oder dem Liposom unter Bedingungen, die dafür ausreichend sind, um die DNA-Sequenz, die Pre-Prodynorphin oder Pre-Prodynorphin-Varianten kodiert, in die Zelle einzubringen.

13. Verabreichungsvektor oder rekombinantes Viruspartikel oder Liposom nach einem der Ansprüche 1 bis 11 zur Verwendung als Arzneimittel.

14. Verabreichungsvektor oder rekombinantes Viruspartikel oder Liposom nach einem der Ansprüche 1 bis 11 und 13 zur Verwendung bei der Behandlung fokaler Epilepsie bei einem Subjekt, insbesondere mesiale Temporallappenepilepsie, oder zur Verwendung bei der Prävention epileptischer Anfälle bei einem Subjekt, das an fokaler Epilepsie leidet, wobei der Verabreichungsvektor oder das rekombinante Viruspartikel oder das Liposom die Aktivierung von menschlichen Kappa-Opioidrezeptoren im epileptogenen Fokus bewirken, womit Anfälle gehemmt werden.

15. Verabreichungsvektor oder rekombinantes Viruspartikel oder Liposom nach einem der Ansprüche 1 bis 11 und 13 zur Verwendung bei der Behandlung fokaler Epilepsie bei einem Subjekt, insbesondere mesiale Temporallappenepilepsie, oder zur Verwendung bei der Prävention epileptischer Anfälle bei einem Subjekt, das an fokaler Epilepsie leidet, wobei der Verabreichungsvektor oder das rekombinante Viruspartikel oder das Liposom die Aktivierung von menschlichen Kappa-Opioidrezeptoren im epileptogenen Fokus bewirken, womit Anfälle gehemmt werden, wobei der Verabreichungsvektor oder das rekombinante Viruspartikel oder das Liposom zur Freisetzung-auf-Anforderung von Peptiden mit agonistischen Effekten auf menschliche Kappa-Opioidrezeptoren im epileptogenen Fokus führt.

16. Verabreichungsvektor oder rekombinantes Viruspartikel oder Liposom nach einem der Ansprüche 1 bis 11 und 13 zur Verwendung bei der Behandlung fokaler Epilepsie bei einem Subjekt, insbesondere mesiale Temporallappenepilepsie, oder zur Verwendung bei der Prävention epileptischer Anfälle bei einem Subjekt, das an fokaler Epilepsie leidet nach Anspruch 13-14, wobei der Vektor oder das rekombinante Viruspartikel zur peripheren Verabreichung oder zur intrakraniellen oder intrazerebralen oder intrathekalen oder intraparenchymalen Verabreichung geeignet ist.

17. Verabreichungsvektor oder rekombinantes Viruspartikel oder Liposom nach einem der Ansprüche 1 bis 11 und 13 zur Verwendung bei der Behandlung fokaler Epilepsie bei einem Subjekt, insbesondere mesiale Temporallappenepilepsie, oder zur Verwendung bei der Prävention epileptischer Anfälle bei einem Subjekt, das an fokaler Epilepsie leidet nach Anspruch 13-14, wobei der Verabreichungsvektor oder das rekombinante Viruspartikel oder das Liposom intrazerebral, vorzugsweise fokal verabreicht wird.

18. Pharmazeutische Freisetzung-auf-Anforderung-Zusammensetzung, umfassend einen Verabreichungsvektor oder ein rekombinantes Viruspartikel oder Liposom nach einem der Ansprüche 1 bis 11 und 13, und optional einen pharmazeutisch verträglichen Trägerstoff.

19. Verabreichungsvektor oder rekombinantes Viruspartikel wie in den Ansprüchen 1 bis 11 und 13 definiert, oder eine pharmazeutische Zusammensetzung nach Anspruch 18 zur Verwendung bei der Behandlung fokaler Epilepsie bei einem Subjekt, insbesondere mesiale Temporallappenepilepsie, oder zur Verwendung bei der Prävention epileptischer Anfälle bei einem Subjekt, das an fokaler Epilepsie leidet,
wobei vorzugsweise der Verabreichungsvektor oder das rekombinante Viruspartikel oder das Liposom Pre-Propeptide kodiert, welche nach Reifung und Freisetzung die Aktivierung von menschlichen Kappa-Opioidrezeptoren im epileptogenen Fokus bewirken, womit Anfälle gehemmt werden, und wobei vorzugsweise der Verabreichungsvektor oder das rekombinante Viruspartikel oder das Liposom intrazerebral, bevorzugt fokal verabreicht wird.

## Revendications

1. Vecteur d'administration comprenant une séquence d'ADN codant pour la pré-prodynorphine ou des variants de la pré-prodynorphine, le vecteur d'administration induisant l'expression d'un pré-propeptide qui permet la libération de dynorphine ou de variants de la dynorphine à la demande, le pré-propeptide étant la pré-prodynorphine ou un variant de la pré-prodynorphine, et ledit pré-propeptide comprenant un peptide signal qui permet l'empaquetage dudit propeptide dans des vésicules, et ledit vecteur d'administration entraînant la libération à la demande de dynorphines ou de variants de dynorphine, ladite pré-prodynorphine ou lesdits variants de pré-prodynorphine comprenant au moins l'une des séquences suivantes choisies dans le groupe:
a. Dyn A qui est SEQ ID n° 7 (AA 207-223 de SEQ ID n° 1; ppDyn) ou un variant de celui-ci constitué des 13 premiers AA (à partir de l'extrémité N-terminale, soit YGGFLRRIRPKLK (SEQ ID n° 16)) ou un variant de celui-ci constitué des 8 premiers AA (à partir de l'extrémité N-terminale, soit YGGFLRRI (SEQ ID n° 17));
b. Dyn B qui est SEQ ID n° 8 (AA 226-238 de SEQ ID n° 1; ppDyn);
c. leumorphine qui est SEQ ID n° 9 (AA 226-254 de SEQ ID n° 1; ppDyn);
d. variants de Dyn A selon SEQ ID n° 7 ayant une identité de séquence d'acides aminés d'au moins 60 % dans les 8 premiers acides aminés comptés à partir de l'extrémité N-terminale de SEQ ID n° 7 (YGGFLRRI (SEQ ID n° 17)), c'est-à-dire ayant une identité de séquence d'acides aminés d'au moins 60 % dans la séquence YGGFLRRI comprise dans SEQ ID n° 7;
e. variants de Dyn B selon SEQ ID n° 8 ayant une identité de séquence d'acides aminés d'au moins 60 % dans les 8 premiers acides aminés comptés à partir de l'extrémité N-terminale de SEQ ID n° 8 (YGGFLRRQ (SEQ ID n° 18)), c'est-à-dire ayant une identité de séquence d'acides aminés d'au moins 60 % dans la séquence YGGFLRRQ comprise dans SEQ ID n° 8;
f. variants de la leumorphine selon SEQ ID n° 9 ayant une identité de séquence d'acides aminés d'au moins 60 % dans les 8 premiers acides aminés comptés à partir de l'extrémité N-terminale de SEQ ID n° 9 (YGGFLRRQ (SEQ ID n° 18)), c'est-à-dire ayant une identité de séquence d'acides aminés d'au moins 60 % dans la séquence YGGFLRRQ comprise dans SEQ ID n° 9.

2. Vecteur d'administration selon la revendication 1, ledit vecteur d'administration entraînant la libération à la demande de dynorphines ou de variants de dynorphines ayant des effets agonistes sur les récepteurs opioïdes kappa humains.

3. Vecteur d'administration selon la revendication 1 ou la revendication 2, les variants ayant une identité de séquence d'acides aminés d'au moins 70 % dans les 8 premiers acides aminés comptés à partir de l'extrémité N-terminale de SEQ ID n° 7 (YGGFLRRI), SEQ ID n° 8 (YGGFLRRQ) ou SEQ ID n° 9 (YGGFLRRQ), respectivement.

4. Vecteur d'administration selon les revendications 1 à 3, les variants ayant une identité de séquence d'acides aminés d'au moins 80 % dans les 8 premiers acides aminés comptés à partir de l'extrémité N-terminale de SEQ ID n° 7, SEQ ID n° 8 ou SEQ ID n° 9, respectivement.

5. Vecteur d'administration selon l'une des revendications 1 à 4, ledit vecteur d'administration induisant l'expression d'un pré-propeptide qui est une pré-prodynorphine ou un variant de pré-prodynorphine, ledit pré-propeptide comprenant un peptide signal, et ledit vecteur d'administration comprenant une séquence d'ADN codant pour un variant de pré-prodynorphine qui comprend au moins l'une des séquences de variants suivantes choisies dans le groupe:
a. SEQ ID n° 10 (YGZFLRRZRPKLKWDNQ),
b. SEQ ID n° 11 (YGZFLRRZFKVVT),
c. SEQ ID n° 12 (YGZFLRRZFKVVTRSQEDPNAYSGELFDA),
où Z représente un quelconque acide aminé naturel, et où au moins un Z dans une séquence selon a., b. ou c. est de préférence substitué par un autre acide aminé par rapport à la séquence de type sauvage dudit fragment de dynorphine selon une séquence selon a., b. ou c.

6. Vecteur d'administration selon l'une des revendications 1 à 5, ledit vecteur d'administration induisant l'expression d'un pré-propeptide qui est une pré-prodynorphine ou un variant de pré-prodynorphine, ledit pré-propeptide comprenant un peptide signal, et ledit vecteur d'administration comprenant une séquence d'ADN codant pour un variant de pré-prodynorphine, où « Z » est choisi dans le groupe comprenant l'alanine, la glycine, l'asparagine, la glutamine, la leucine, la sérine, la valine et l'isoleucine.

7. Vecteur d'administration selon l'une des revendications 1 à 6, ledit vecteur d'administration comprenant en outre un génome de vecteur de virus adéno-associé recombinant (AAV) ou un génome de lentivirus recombinant.

8. Vecteur d'administration selon l'une des revendications 1 à 7, comprenant un génome de vecteur viral adéno-associé recombinant (AAV), ledit vecteur étant un vecteur de sérotype humain choisi dans le groupe comprenant les sérotypes 1, 2, 3, 4, 5, 6, 7, 8, 9, 10, rh10, 11, 12, 13, 14, l'AAV serpentine, l'AAV ancestral ou des mutants de capside d'AAV qui en sont dérivés, de préférence du sérotype 1 ou 2.

9. Vecteur d'administration selon les revendications 1 à 8, ledit vecteur d'administration induisant l'expression d'un pré-propeptide qui est une pré-prodynorphine ou un variant de pré-prodynorphine, ledit pré-propeptide comprenant un peptide signal, et ledit vecteur d'administration comprenant une séquence d'ADN codant pour un variant de pré-prodynorphine qui comprend au moins l'une des séquences suivantes de variants choisies dans le groupe comprenant les peptides ayant les numéros d'identification SEQ ID n° 10, SEQ ID n° 11, SEQ ID n° 12, SEQ ID n° 13, SEQ ID n° 14 et SEQ ID n° 15.

10. Particule virale recombinante ou liposome, comprenant un vecteur d'administration selon l'une des revendications précédentes.

11. Particule virale recombinante ou liposome selon la revendication 10, ledit vecteur d'administration comprenant en outre un génome de vecteur viral adéno-associé recombinant (AAV) et ledit génome de vecteur rAAV étant encapsidé dans une capside AAV ou ledit vecteur d'administration comprenant en outre un génome de vecteur lentiviral recombinant et étant encapsidé dans une particule lentivirale.

12. Vecteur d'administration ou particule virale recombinante ou liposome selon l'une des revendications 1 à 11, destiné à être utilisé pour livrer un acide nucléique à une cellule du système nerveux central, comprenant la mise en contact de la cellule avec le vecteur d'administration ou la particule virale recombinante ou le liposome dans des conditions suffisantes pour que la séquence d'ADN codant pour la pré-prodynorphine ou des variants de la pré-prodynorphine soit introduite dans la cellule.

13. Vecteur d'administration ou particule virale recombinante ou liposome selon l'une des revendications 1 à 11, destiné à être utilisé comme médicament.

14. Vecteur d'administration ou particule virale recombinante ou liposome selon l'une des revendications 1 à 11 et 13, destiné à être utilisé dans le traitement de l'épilepsie focale chez un sujet, en particulier l'épilepsie mésiale du lobe temporal, ou destiné à être utilisé dans la prévention de crises d'épilepsie chez un sujet souffrant d'épilepsie focale, ledit vecteur d'administration ou particule virale recombinante ou liposome provoquant l'activation des récepteurs opioïdes kappa humains dans le foyer épileptogène, inhibant ainsi les crises.

15. Vecteur d'administration ou particule virale recombinante ou liposome selon l'une des revendications 1 à 11 et 13, destiné à être utilisé dans le traitement de l'épilepsie focale chez un sujet, en particulier l'épilepsie mésiale du lobe temporal, ou destiné à être utilisé dans la prévention de crises d'épilepsie chez un sujet souffrant d'épilepsie focale, ledit vecteur d'administration ou particule virale recombinante ou liposome assurant l'activation des récepteurs opioïdes kappa humains dans le foyer épileptogène, inhibant ainsi les crises, ledit vecteur d'administration ou ladite particule virale recombinante ou ledit liposome entraînant la libération à la demande de peptides ayant des effets agonistes sur les récepteurs opioïdes kappa humains dans le foyer épileptogène.

16. Vecteur d'administration ou particule virale recombinante ou liposome selon l'une des revendications 1 à 11 et 13, destiné à être utilisé dans le traitement de l'épilepsie focale chez un sujet, en particulier l'épilepsie mésiale du lobe temporal, ou destiné à être utilisé dans la prévention de crises épileptiques chez un sujet souffrant d'épilepsie focale selon les revendications 13-14, ledit vecteur ou ladite particule virale recombinante étant adapté(e) à une administration périphérique ou intracrânienne ou intracérébrale ou intrathécale ou intraparenchymateuse.

17. Vecteur d'administration ou particule virale recombinante ou liposome selon l'une des revendications 1 à 11 et 13, destiné à être utilisé dans le traitement de l'épilepsie focale chez un sujet, en particulier l'épilepsie mésiale du lobe temporal, ou destiné à être utilisé dans la prévention de crises épileptiques chez un sujet souffrant d'épilepsie focale selon les revendications 13-14, ledit vecteur d'administration ou ladite particule virale recombinante ou ledit liposome étant appliqué de manière intracérébrale, de préférence de manière focale.

18. Composition pharmaceutique à libération à la demande comprenant un vecteur d'administration ou une particule virale recombinante ou un liposome selon l'une des revendications 1 à 11 et 13, et éventuellement un support pharmaceutiquement acceptable.

19. Vecteur d'administration ou particule virale recombinante selon les revendications 1 à 11 et 13, ou composition pharmaceutique selon la revendication 18, destiné à être utilisé dans le traitement de l'épilepsie focale chez un sujet, en particulier l'épilepsie mésiale du lobe temporal, ou destiné à être utilisé dans la prévention des crises d'épilepsie chez un sujet souffrant d'épilepsie focale,
où, de préférence, ledit vecteur d'administration ou ladite particule virale recombinante ou ledit liposome code pour des pré-propeptides qui, après maturation et libération, assurent l'activation des récepteurs opioïdes kappa humains dans le foyer épileptogène, inhibant ainsi les crises, et où, de préférence, ledit vecteur d'administration ou ladite particule virale recombinante ou ledit liposome est appliqué par voie intracérébrale, de préférence de manière focale.
